# EUROPEAN PATENT APPLICATION

(11) **EP 4 779 018 A1**
(43) Date of publication of application: **22.07.2026**
(21) Application number: 26152317.9
(22) Date of filing: 16.01.2026
(51) Int. Cl.: C12N 5/0784, C07K 14/705

(54) **DENDRITIC CELL AND PREPARATION METHOD AND APPLICATION THEREOF**

(30) Priority: 17.01.2025 CN 202510075003
(71) Applicant: Beijing DCT Cell Technology Co., Ltd., Beijing City 100176 (CN)
(72) Inventor: TIAN, Guangqi, Beijing City, 100176 (CN); FU, Hanjiang, Beijing City, 100176 (CN); LI, Xiaozhu, Beijing City, 100176 (CN); LEI, Shasha, Beijing City, 100176 (CN)
(74) Representative: karo IP

(57) **Abstract**

This application discloses a dendritic cell (DC) and a preparation method and application thereof, belonging to the technical field of immune cells. The DC is a DC that does not express HLA-I class molecules, obtained by knocking out HLA-I class genes in a starting DC. In its application, according to an HLA-I class molecule subtype of a target antigen-specific T cell to be obtained, HLA-I class molecules that match the target antigen-specific T cells can be re-expressed in the DC through gene editing, viral, or mRNA techniques, thereby overcoming the HLA-I class molecule-specific matching restriction that exists when using allogeneic DCs, and solving the problem of insufficient HLA-I class molecule subtype coverage of a single DC.

## Description

### Technical field

This application belongs to the technical field of immune cells, and specifically relates to a dendritic cell and a preparation method and application thereof.

### Background

The immune system can recognize foreign components and clear potential harmful foreign molecules and cells from the body, and also has the ability to recognize and destroy abnormal cells derived from the host's own tissues. Immune response includes innate immunity and adaptive immunity, wherein, adaptive immunity includes humoral immunity by B cell responses (B cells develop into plasma cells and secrete soluble antigen-specific antibodies) and cell-mediated immunity mediated by specific subgroups of T cells. T cell-dependent (T cell-mediated) adaptive immune responses generally require antigen-presenting cells (APCs) to present antigen peptides bound to major histocompatibility complex (MHC) molecules to T cells. In humans, MHC is also referred to as the human leukocyte antigen (HLA) complex.

Dendritic cells (DCs) are specialized antigen-presenting cells that participate in both cell immunity and humoral immunity, and are a key node of the entire immune system having enormous influence on immune system function. One of the main roles of a DC is to recognize and present a captured antigen to a naive T lymphocyte, stimulating T cell activation and proliferation, generating large numbers of T lymphocytes that recognize the specific antigen and possess strong cytotoxicity, namely cytotoxic T lymphocytes (CTL cells). Activation of T cells requires CD4 or CD8 on the T cell surface to bind to an HLA molecule, as well as the alpha and beta peptide chains on the T cell receptor (TCR) on the T cell surface to mutually recognize and mutually interact with the antigen-MHC complex.

HLA molecules used to present antigens mainly include two classes: HLA-I class molecules and HLA-II class molecules. Wherein, HLA-I class molecules mainly include three types (HLA-A molecule, HLA-B molecule, and HLA-C molecule), and the genes encoding these three molecules are the HLA-A gene, HLA-B gene, and HLA-C gene, respectively. CD8 T cells mainly react with HLA-I class molecules, subsequently becoming activated and proliferating, generating large numbers of T lymphocytes that recognize specific antigens and possess strong cytotoxicity.

HLA genes are located on human chromosome 6 and constitute the most complex and highly polymorphic genetic system known in human populations, possessing extremely large numbers of alleles. This results in HLA molecules having highly polymorphic characteristics, and HLA molecules of different individuals exhibit large differences in structure and composition. This is also an important reason for immune reactions occurring during organ transplantation, because HLA molecules on donor-organ cell surfaces are foreign antigens to the recipient's immune system. When an antigen captured by an allogeneic-source DC is presented to activate T cells between different individuals, the same issue exists; the HLA molecules of the recipient's T cells do not match the HLA molecules of the donor DC, causing CD4 or CD8 on the surface of the recipient's T cells to be unable to bind to the HLA molecules on the surface of the donor's DC, and the TCR cannot mutually recognize or mutually interact with the antigen-MHC complex, thereby failing to achieve activation of the target T cells; that is, there is an HLA molecule-specific matching restriction.

In 1996, Hsu FJ et al. from the Oncology Department of Stanford University Medical Center first reported clinical trials of dendritic cell vaccines in Nature Medicine. Research on DC tumor vaccines worldwide has continued for decades, and multiple DC tumor vaccines have now received market approval, such as Sipuleucel-T (Provenge), CreaVax RCC, Hybricell, and DCVax Brain. Traditional DC immunotherapy first collects peripheral blood mononuclear cells (PBMCs) from a patient, which undergoes a series of induced cultures to differentiate into mature DCs; then, the DCs are loaded in vitro with tumor or viral antigens; then, they are infused back into the patient, or after antigen-specific T cells are incited and activated in vitro, the activated antigen-specific T cells are infused.

In traditional treatment methods, the source of mature DCs and the differentiation processes are limited, time-consuming, and labor-intensive. Moreover, due to HLA molecule-specific matching restriction, only autologous PBMCs can be used for induced differentiation, which increases the burden for individuals already in poor health.

Therefore, a DC immunotherapy capable of overcoming the HLA molecule-specific matching restriction, particularly overcoming the HLA-I class molecule-specific matching restriction, has extremely important significance.

### Summary

### 1. Problems to be solved

To solve the above-described problem in the prior art of allogeneic-source DCs having HLA-I class molecule-specific matching restriction during application, this application provides a DC and a preparation method and application thereof. By knocking out the HLA-I class genes (HLA-A gene, HLA-B gene, and/or HLA-C gene) in a starting DC, a DC that does not express the HLA-I class molecules is obtained; during application, according to the HLA-I class molecule subtype of the target antigen-specific T cells to be obtained, the HLA-I class molecule subtype that matches the target antigen-specific T cells is re-expressed in the DC through gene editing, viral, or mRNA techniques, thereby overcoming the HLA-I class molecule-specific matching restriction that exists during application of allogeneic-source DCs, and also solving the problem of insufficient coverage of a single DC HLA-I class molecule subtype.

### 2. Technical solutions

In order to solve the above-described problems, the technical solutions adopted by this application are as follows:
In a first aspect, this application provides a dendritic cell (DC), HLA-I class genes of the DC being knocked out, the HLA-I class genes including an HLA-A gene, an HLA-B gene, and/or an HLA-C gene. This DC overcomes the restriction of HLA-I class molecule-specific matching, that is: the DC (starting DC) before knockout of the HLA-I class genes is only able to incite activation of antigen-specific T cells that match its own HLA-A molecule, HLA-B molecule, and/or HLA-C molecule subtype; in contrast, the DC with the HLA-I class genes knocked out can, by reintroducing a new HLA-A gene, HLA-B gene, and/or HLA-C gene, incite antigen-specific T cells that match the new HLA-A molecule, HLA-B molecule, and/or HLA-C molecule subtype. Although "HLA-I class genes" include three types (HLA-A gene, HLA-B gene, and HLA-C gene), those skilled in the art understand that the restriction of molecule-specific matching is mainly a type of gene (subtype) restriction. Therefore, "HLA-I class genes" as used herein may refer to any one of, or any combination of, the HLA-A gene, HLA-B gene, and HLA-C gene. For example, "HLA-I class genes" herein may refer to the HLA-A gene, HLA-B gene, HLA-C gene, HLA-A gene and HLA-B gene, HLA-B gene and HLA-C gene, HLA-A gene and HLA-C gene, or HLA-A gene, HLA-B gene, and HLA-C gene.

In some specific embodiments of this application, the HLA-A gene of the above-described DC is knocked out, the DC having the HLA-A molecule-specific matching restriction removed. That is, the DC (starting DC) before knockout of the HLA-A gene is only able to incite activation of antigen-specific T cells that are the same (match) its own HLA-A molecule subtype, whereas the DC with the HLA-A gene knocked out can, by reintroducing a new HLA-A gene, incite activation of antigen-specific T cells that match the new HLA-A molecule subtype.

In some specific embodiments of this application, the HLA-B gene of the above-described DC is knocked out, the DC having the HLA-B molecule-specific matching restriction removed.

In some specific embodiments of this application, the HLA-C gene of the above-described DC is knocked out, the DC having the HLA-C molecule-specific matching restriction removed.

In some specific embodiments of this application, the HLA-A gene and HLA-B gene of the above-described DC are knocked out, the DC having the HLA-A molecule- and HLA-B molecule-specific matching restriction removed. That is, the DC (starting DC) before knockout of the HLA-A gene and HLA-B gene is only able to incite activation of antigen-specific T cells that are the same (match) its own HLA-A molecule and/or HLA-B molecule subtype, whereas the DC with the HLA-A gene and/or HLA-B gene knocked out can, by reintroducing a new HLA-A gene and/or HLA-B gene, incite activation of antigen-specific T cells that match the new HLA-A molecule and/or second HLA-B molecule subtype.

In some specific embodiments of this application, the HLA-A gene and HLA-C gene of the above-described DC are knocked out, the DC having the HLA-A molecule- and HLA-C molecule-specific matching restriction removed.

In some specific embodiments of this application, the HLA-B gene and HLA-C gene of the above-described DC are knocked out, the DC having the HLA-B molecule- and HLA-C molecule-specific matching restriction removed.

In some specific embodiments of this application, the HLA-A gene, HLA-B gene, and HLA-C gene of the above-described DC are knocked out, the DC having the HLA-A molecule-, HLA-B molecule- and HLA-C molecule-specific matching restriction removed. Reintroducing a new HLA-A gene, HLA-B gene, and/or HLA-C gene can incite activation of antigen-specific T cells that match the new HLA-A molecule, HLA-B molecule, and/or HLA-C molecule subtype.

In some specific embodiments of this application, the above-described DC is an immortalized DC. The immortalized DC can be obtained by knocking out the HLA-I class genes of the immortalized DC. Immortalized cells can proliferate indefinitely in vitro, thereby meeting the quantity and activity requirements of DCs, thereby able to overcome the limitations of scarce quantity and difficulty of in vitro culture and expansion of primary DCs; at the same time, the problems of low quantity and high cost of monocyte-derived DCs (MODCs) can be overcome.

In some specific embodiments of this application, the above-described immortalized DC is UDC981, deposited at China General Microbiological Culture Collection Center (CGMCC), with a deposit time of December 25, 2024, deposit address of No. 3, Yard 1, Beichen West Road, Chaoyang District, Beijing, and deposit number of CGMCC No. 46260.

In a second aspect, this application further provides a cell population of a DC, the cell population including any one of the above-described DCs.

In a third aspect, this application further provides a cell line of a DC, the cell line including any one of the above-described DCs.

In a fourth aspect, this application further provides a preparation method for a type of the above-described DC, the preparation method including the following steps:
knocking out HLA-I class genes of a starting DC, the HLA-I class genes including an HLA-A gene, an HLA-B gene, and/or an HLA-C gene;
the DC screened for negative expression of the HLA-I class genes is the DC.

In some specific embodiments of this application, the HLA-A gene of the starting DC is knocked out; DCs screened for negative expression of the HLA-A gene are the DCs in which the HLA-A molecule-specific matching restriction has been removed.

In some specific embodiments of this application, the HLA-B gene of the starting DC is knocked out; DCs screened for negative expression of the HLA-B gene are the DCs in which the HLA-B molecule-specific matching restriction has been removed.

In some specific embodiments of this application, the HLA-C gene of the starting DC is knocked out; DCs screened for negative expression of the HLA-C gene are the DCs in which the HLA-C molecule-specific matching restriction has been removed.

In some specific embodiments of this application, the HLA-A gene and HLA-B gene of the starting DC are knocked out; DCs screened for negative expression of the HLA-A gene and HLA-B gene are the DCs in which the HLA-A molecule- and HLA-B molecule-specific matching restriction has been removed.

In some specific embodiments of this application, the HLA-A gene and HLA-C gene of the starting DC are knocked out; DCs screened for negative expression of the HLA-A gene and HLA-C gene are the DCs in which the HLA-A molecule- and HLA-C molecule-specific matching restriction has been removed.

In some specific embodiments of this application, the HLA-B gene and HLA-C gene of the starting DC are knocked out; DCs screened for negative expression of the HLA-B gene and HLA-C gene are the DCs in which the HLA-B molecule- and HLA-C molecule-specific matching restriction has been removed.

In some specific embodiments of this application, the HLA-A gene, HLA-B gene, and HLA-C gene of the starting DC are knocked out; DCs screened for negative expression of the HLA-A gene, HLA-B gene, and HLA-C gene are the DCs in which the HLA-A molecule-, HLA-B molecule-, and HLA-C molecule-specific matching restriction has been removed.

In some specific embodiments of this application, the method for knocking out the HLA-I class genes of the starting DC includes: homologous recombination, CRISPR/Cas gene editing, or TALEN (transcription activator-like effector nuclease).

In some specific embodiments of this application, the CRISPR/Cas gene editing method includes:
designing one or more sgRNA that bind to the HLA-I class genes of the starting DC; in this application, "designing one or more sgRNA that bind to the HLA-I class genes of the starting DC" means that the HLA-I class genes for the sgRNA to bind to can be determined according to the DC intended to be prepared; if the DC is a DC in which the HLA-A gene is knocked out, at least the sgRNA that binds to the HLA-A gene of the starting DC is designed; if the DC is a DC in which the HLA-A gene and HLA-B gene are knocked out, at least two sgRNAs that respectively bind to the HLA-A gene and HLA-B gene can be designed, or one sgRNA that simultaneously binds to at least the HLA-A gene and HLA-B gene can be designed;
using a CRISPR-Cas system to introduce the sgRNA into the starting DC to knock out the HLA-I class genes.

In some specific embodiments of this application, the above-described CRISPR-Cas system includes a CRISPR-Cas9 system or a CRISPR-Cas13 system.

In some specific embodiments of this application, the above-described sgRNA binds to one or more HLA-I class genes.

In some specific embodiments of this application, the above-described sgRNA includes 17-23 bases.

In some specific embodiments of this application, the HLA-A gene subtype of the above-described starting DC is HLA-A0201 and/or HLA-A6801; and/or the HLA-B gene subtype is HLA-B0801 and/or HLA-B 1507; and/or the HLA-C gene subtype is HLA-C0303 and/or HLA-C0702.

In some specific embodiments of this application, a nucleotide sequence of the sgRNA that binds to the HLA-I class genes of the above-described starting DC is as shown in SEQ ID NO. 1. This sgRNA can simultaneously target a pair of alleles of the HLA-A gene (HLA-A0201 and HLA-A6801), a pair of alleles of the HLA-B gene (HLA-B0801 and HLA-B1507), and a pair of alleles of the HLA-C gene (HLA-C0303 and HLA-C0702) of the above-described starting DC, thereby simultaneously knocking out three pairs of alleles with extremely high gene-editing efficiency.

In some specific embodiments of this application, the above-described starting DC is an immortalized DC. The immortalized DC can be obtained by knocking out the HLA-I class genes of the immortalized DC. The immortalized DC can proliferate indefinitely in vitro, thereby meeting the quantity and activity requirements of DCs, thereby able to overcome the limitations of scarce quantity and difficulty of in vitro culture and expansion of primary DCs; at the same time, the problems of low quantity and high cost of MODCs can be overcome.

In some specific embodiments of this application, the above-described immortalized DC is DC0502, deposited at China General Microbiological Culture Collection Center (CGMCC), with a deposit time of December 25, 2024, deposit address of No. 3, Yard 1, Beichen West Road, Chaoyang District, Beijing, and deposit number of CGMCC No. 46259. The HLA-A gene subtype of the immortalized DC is HLA-A0201 and HLA-A6801; the HLA-B gene subtype is HLA-B0801 and HLA-B 1507; the HLA-C gene subtype is HLA-C0303 and HLA-C0702.

In a fifth aspect, this application further provides an application of the above-described DC in activating target antigen-specific T cells, the application including:
Obtaining an HLA-I class molecule subtype of a T cell population that is restricted by a target antigen, the HLA-I class molecule including an HLA-A molecule, an HLA-B molecule, and/or an HLA-C molecule;
Introducing an HLA-I class gene encoding the above-described HLA-I class molecule subtype into the DC with *HLA-I* class genes knocked out; when reintroducing the HLA-I class gene, if the HLA-I class molecule subtype encoded by the HLA-I class gene is different from the original HLA-I class molecule subtype of the DC, then the HLA-I class molecule-specific matching restriction of the starting DC is overcome; or, a gene encoding the same HLA-I class molecule subtype can be introduced for activating autologous T cells;
Loading the DCs introduced with the HLA-I class gene with an antigen peptide that is restricted to the HLA-I class molecule subtype, an antigen epitope of the antigen peptide being an antigen epitope recognized by the target antigen-specific T cell;
Co-culturing the DCs loaded with the antigen peptide with the above-described T cell population, thereby activating specific T cells capable of recognizing the above-described antigen epitope.

In some specific embodiments of this application, the above-described method of introducing the HLA-I class gene encoding the above-described HLA-I class molecule includes: gene editing, viral, or mRNA methods.

In some specific embodiments of this application, the above-described antigen peptide is derived from a tumor or a virus.

In some specific embodiments of this application, the above-described tumor includes melanoma.

In some specific embodiments of this application, the above-described virus includes cytomegalovirus.

In some specific embodiments of this application, the above-described T cell population includes PBMC.

In a sixth aspect, this application further provides an application of the above-described DC cell population in the activation of target antigen-specific T cells, the DC cell population including the above-described DC.

In a seventh aspect, this application further provides an application of the above-described DC cell line in the activation of target antigen-specific T cells, the DC cell line including the above-described DC.

In an eighth aspect, this application further provides an application of the above-described DC in the prevention or treatment of tumors or viral infections, the application including: administering into a subject an effective amount of DC cells loaded with tumor or viral antigen peptides; the DC cells have been introduced with a gene encoding the same HLA-I class molecule subtype as that of the subject, and the antigen peptide is an antigen peptide restricted to the HLA-I class molecule subtype.

In a ninth aspect, this application further provides an application of the above-described DC cell population in the prevention or treatment of tumors or viral infections.

In a tenth aspect, this application further provides an application of the above-described DC cell line in the prevention or treatment of tumors or viral infections.

In an eleventh aspect, this application further provides a method for expanding target antigen-specific T cells, the method including:
Obtaining an HLA-I class molecule subtype of a T cell population that is restricted by a target antigen, the HLA-I class molecule including an HLA-A molecule, an HLA-B molecule, and/or an HLA-C molecule;
Introducing an HLA-I class gene encoding the above-described HLA-I class molecule subtype into the DC with *HLA-I* class genes knocked out;
Loading the DCs introduced with the HLA-I class gene with an antigen peptide that is restricted to the HLA-I class molecule subtype, an antigen epitope of the antigen peptide being an antigen epitope recognized by the target antigen-specific T cell;
Co-culturing the DCs loaded with the antigen peptide with the above-described T cell population, thereby activating and expanding specific T cells capable of recognizing the above-described antigen epitope.

In some specific embodiments of this application, the above-described method of introducing the HLA-I class gene encoding the above-described HLA-I class molecule includes: gene editing, viral, or mRNA methods.

In some specific embodiments of this application, the above-described antigen peptide is derived from a tumor or a virus.

In some specific embodiments of this application, the above-described tumor includes melanoma.

In some specific embodiments of this application, the above-described virus includes cytomegalovirus.

In some specific embodiments of this application, the above-described T cell population includes PBMC.

In a twelfth aspect, this application further provides an application of the above-described DC in the preparation of a reagent for expanding target antigen-specific T cells.

In a thirteenth aspect, this application further provides an application of the above-described DC cell population in the preparation of a reagent for expanding target antigen-specific T cells.

In a fourteenth aspect, this application further provides an application of the above-described DC cell line in the preparation of a reagent for expanding target antigen-specific T cells.

In some specific embodiments of this application, the above-described reagent further includes an HLA-I class gene encoding an HLA-I class molecule subtype of the above-described target antigen-specific T cells, the HLA-I class molecule subtype including an HLA-A molecule, an HLA-B molecule, and/or an HLA-C molecule.

In a fifteenth aspect, this application further provides a modified DC, original HLA-I class genes in the modified DC being knocked out, and at the same time having exogenous HLA-I class genes introduced, the exogenous HLA-I class genes being HLA-I class genes of a single subtype, the exogenous HLA-I class genes including an HLA-A gene, an HLA-B gene, or an HLA-C gene; the original HLA-I class genes at least include the same type of HLA-I class gene as the exogenous HLA-I class gene. The applicant has found through research that DCs introduced with HLA-I class genes of a single subtype have a stronger ability to activate and expand antigen-specific CD8 T cells compared with the starting DCs, presumably because the HLA-I class genes of the starting DCs typically contain two allele subtypes, whereas the introduced exogenous HLA-I class genes only have one subtype, thereby possessing a stronger ability to activate and expand antigen-specific CD8 T cells.

In a sixteenth aspect, this application further provides a preparation method for the above-described modified DC, the method including:
Introducing an HLA-I class gene of a single subtype into the above-described DC with *HLA-I* class genes knocked out to obtain the modified DC.

In a seventeenth aspect, this application further provides an sgRNA, a nucleotide sequence of the sgRNA being as shown in SEQ ID NO. 1; the sgRNA is capable of simultaneously targeting the pair of alleles HLA-A0201 and HLA-A6801 of the HLA-A gene, the pair of alleles HLA-B0801 and HLA-B1507 of the HLA-B gene, and the pair of alleles HLA-C0303 and HLA-C0702 of the HLA-C gene, and can be used for simultaneously knocking out the HLA-A, HLA-B, and HLA-C genes of cells containing the above-described gene subtypes.

In an eighteenth aspect, this application further provides an application of the above-described sgRNA in knocking out the HLA-I class genes of cells, the HLA-I class genes including HLA-A, HLA-B, and/or HLA-C; the HLA-A gene subtype of the cell is HLA-A0201 and/or HLA-A6801; and/or the HLA-B gene subtype is HLA-B0801 and/or HLA-B1507; and/or the HLA-C gene subtype is HLA-C0303 and/or HLA-C0702.

In some specific embodiments of this application, in the application of the above-described sgRNA in knocking out the HLA-I class genes of cells, the HLA-I class genes include HLA-A, and the HLA-A gene subtype of the cells is HLA-A0201 and/or HLA-A6801.

In some specific embodiments of this application, in the application of the above-described sgRNA in knocking out the HLA-I class genes of cells, the HLA-I class genes include HLA-B, and the HLA-B gene subtype of the cells is HLA-B0801 and/or HLA-B1507.

In some specific embodiments of this application, in the application of the above-described sgRNA in knocking out the HLA-I class genes of cells, the HLA-I class genes include HLA-C, and the HLA-C gene subtype of the cells is HLA-C0303 and/or HLA-C0702.

In some specific embodiments of this application, in the application of the above-described sgRNA in knocking out the HLA-I class genes of cells, the HLA-I class genes include HLA-A, HLA-B, and HLA-C, and the HLA-A gene subtype of the cells is HLA-A0201 and/or HLA-A6801, the HLA-B gene subtype is HLA-B0801 and/or HLA-B1507; and the HLA-C gene subtype is HLA-C0303 and/or HLA-C0702.

In some specific embodiments of this application, in the application of the above-described sgRNA in knocking out the HLA-I class genes of cells, the cells include DCs and/or T cells.

In some specific embodiments of this application, in the application of the above-described sgRNA in knocking out the HLA-I class genes of cells, the cells are DCs.

In some specific embodiments of this application, the above-described DC is an immortalized DC.

In some specific embodiments of this application, the above-described immortalized DC is DC0502, deposited at China General Microbiological Culture Collection Center (CGMCC), with a deposit time of December 25, 2024, deposit address of No. 3, Yard 1, Beichen West Road, Chaoyang District, Beijing, and deposit number of CGMCC No. 46259. The HLA-A gene subtype of the immortalized DC is HLA-A0201 and HLA-A6801; the HLA-B gene subtype is HLA-B0801 and HLA-B 1507; the HLA-C gene subtype is HLA-C0303 and HLA-C0702.

In a nineteenth aspect, this application further provides the immortalized DC DC0502, deposited at China General Microbiological Culture Collection Center (CGMCC), with a deposit time of December 25, 2024, deposit address of No. 3, Yard 1, Beichen West Road, Chaoyang District, Beijing, and deposit number of CGMCC No. 46259.

### 3. Beneficial effects

Compared with the prior art, the beneficial effects of this application are the following:
(1) In the DC and the preparation method and application thereof provided by this application, a DC (UDC) that does not express HLA-I class molecules is obtained by knocking out the HLA-I class genes (HLA-A gene, HLA-B gene, and/or HLA-C gene) of the starting DC, particularly of the immortalized DC. During application, according to the HLA-I class molecule subtype of the target antigen-specific T cells to be obtained, that is, for different individuals, gene editing, viral, or mRNA gene-introducing techniques can be used to re-express in the DC (UDC) an HLA-I class molecule that matches the target antigen-specific T cells, thereby removing the restriction of HLA-I class molecule-specific matching that exists when using allogeneic DCs. This is expected to solve the pain points of personalized cellular immunotherapy products, such as long preparation cycles and excessively high cost, to become an "off-the-shelf" personalized cellular therapy product, greatly reducing cost, and having broad application value for same-species allogeneic off-the-shelf cellular immunotherapy products.
(2) In the DC and the preparation method and application thereof provided by this application, an HLA-I class molecule that matches the target antigen-specific T cells is re-expressed in the DC (UDC). This HLA-I class molecule possesses only one subtype, whereas the HLA-I class alleles of either autologous or allogeneic starting DCs generally possess two subtypes. Compared with the starting DC, interference from a same-type HLA-I class molecule is reduced, improving the ability to activate and expand antigen-specific CD8 T cells.
(3)In the DC and the preparation method and application thereof provided by this application, an HLA-I class molecule that matches the target antigen-specific T cells is re-expressed in the DC (UDC), avoiding non-specific activation and expansion of alloreactive T cells caused by HLA mismatch, thereby reducing the risk of graft-versus-host disease or immune rejection.
(4) In the DC and the preparation method and application thereof provided by this application, when the original cell is an immortalized DC, an immortalized DC that possesses unlimited expansion capacity can be obtained. For example, the monoclonal UDC981 provided by this application reached an expansion fold of 18,000-fold after 30 days of culture, and can be conveniently and rapidly cultured to meet the requirements for DC quantity and activity, thereby able to overcome the limitations of scarce quantity and difficulty of in vitro culture and expansion of primary DCs; at the same time, the problems of low quantity and high cost of MODCs can be overcome.
(5) In the DC and the preparation method and application thereof provided by this application, an sgRNA having a nucleotide sequence as shown in SEQ ID NO. 1 is designed for the immortalized DC DC0502 previously screened by the applicant, which can simultaneously target the pair of alleles HLA-A0201 and HLA-A6801 of the HLA-A gene, the pair of alleles HLA-B0801 and HLA-B1507 of the HLA-B gene, and the pair of alleles HLA-C0303 and HLA-C0702 of the HLA-C gene of DC0502. Combined with genome-editing methods, it can be used to simultaneously knock out the HLA-A gene, HLA-B gene, and HLA-C gene of DC0502 with extremely high gene-editing efficiency.

### Brief description of the drawings

FIGURE 1 is, for this application, flow-cytometry detection results of proportions of HLA-ABC-negative and HLA-A0201-negative cells (left of arrow) after gene editing of the starting DC (DC0502), as well as results of proportions of HLA-ABC-negative and HLA-A0201-negative DCs (UDCs, right of arrow) after magnetic bead negative selection, wherein: "unstain" is the unstained control group; "UDC mix" is the DC population after gene editing.
FIGURE 2 is, for this application, cell morphology photographs of DCs before and after gene editing of this application, wherein: the left photograph is the starting DC (DC0502) without gene editing performed, and the right photograph is the DC (UDC mix) after gene editing.
FIGURE 3 is, for this application, overlaid histograms of flow cytometry analysis of DC monoclonals with a homozygous background, selected from purified, gene-edited DCs in which the three genes HLA-A, HLA-B, and HLA-C (each gene being a pair of alleles) were completely knocked out.
FIGURE 4 is, for this application, statistical results of the mean fluorescence intensity (MFI) of three antibodies in flow cytometry analysis of DC monoclonals with a homozygous background, selected from purified, gene-edited DCs in which the three genes HLA-A, HLA-B, and HLA-C (each gene being a pair of alleles) were completely knocked out.
FIGURE 5 is sequencing results of the three genes HLA-A, HLA-B, and HLA-C (each gene being a pair of alleles) of the monoclonal UDC981 prepared in this application.
FIGURE 6 is detection results of the expansion fold of the starting DC (DC0502) and the prepared monoclonal UDC981 in this application.
FIGURE 7 to FIGURE 9 are flow cytometry results identifying the phenotype and costimulatory molecules of the prepared monoclonal UDC981 in this application, wherein "isotype" refers to the immunoglobulin (Ig) isotype.
FIGURE 10 to FIGURE 12 are flow cytometry results identifying the phenotype and costimulatory molecules of the starting DC (DC0502) used in this application.
FIGURE 13 is, for this application, flow cytometry detection results of HLA-A molecule positivity rates of UDC HLA-A0201, UDC HLA-A1101, and UDC HLA-A2402 after infecting UDC981 with HLA-A0201, HLA-A1101, and HLA-A2402 lentiviruses and after performing magnetic bead positive selection.
FIGURE 14 is, for this application, results validating the ability of UDC981 reintroduced with HLA-A0201 (UDC HLA-A0201) to present the CMV epitope (cytomegalovirus epitope).
FIGURE 15 is, for this application, results validating the ability of UDC981 reintroduced with HLA-A0201 (UDC HLA-A0201) to present the Mart-1 epitope (melanoma epitope).
FIGURE 16 is, for this application, results validating the ability of UDC981 reintroduced with HLA-A2402 (UDC HLA-A2402) to present the CMV epitope (cytomegalovirus epitope).
FIGURE 17 is, for this application, results of immunogenicity testing of UDC981 reintroduced with HLA-A11 (UDC HLA-A11) against allogeneic PBMC.

### Detailed description of embodiments

Below combines specific embodiments to further describe this application.

Unless otherwise defined, all technical and scientific terms used herein have the same meanings as ordinarily understood by those skilled in the technical field to which this application belongs; the term "and/or" used herein includes any and all combinations of one or more of the associated listed items.

For embodiments in which specific conditions are not indicated, they are performed according to conventional conditions or the conditions recommended by the manufacturer. Reagents or instruments for which the manufacturer is not specified are all conventional products obtainable commercially.

As used herein, the term "about" is used to provide flexibility and imprecision associated with a given term, measurement, or value. The degree of flexibility of a specific variable can be readily determined by those skilled in the art.

Concentration, amount, and other numerical data may be presented herein in range format. It should be understood that such range format is used merely for convenience and brevity, and should be interpreted flexibly as including not only the values explicitly stated as the limits of the range, but also all individual values or sub-ranges encompassed within the stated range, as though each individual value and sub-range were explicitly stated. For example, a numerical range of about 1 to about 4.5 should be interpreted as including not only the explicit limits of 1 to about 4.5 but also individual numbers (such as 2, 3, 4) and sub-ranges (such as 1-3, 2-4, etc.). The same principle applies to ranges in which only a single value is stated, such as "less than about 4.5," which should be interpreted as including all above-described values and ranges. Furthermore, such interpretation shall apply regardless of the breadth of the described range or feature.

As used herein, "dendritic cell" (DC) is a professional antigen-presenting cell that participates in both cellular immunity and humoral immunity. In this application, unless otherwise specified, "DC" may represent a cell strain or a cell line. A cell strain refers to a cell population obtained from a primary culture or a cell line through selection or clonal formation methods and possesses special properties or markers, such as UDC981 obtained after gene editing and screening in this application. A cell line refers to a cell population that proliferates after the initial successful passaging of the primary culture, such as DC0502 formed after successful passaging.

As used in this application, "immune system" refers to the system by which an organism performs immune responses and immune functions. It consists of immune organs, immune cells, and immune molecules capable of recognizing and clearing foreign pathogens (such as bacteria, viruses, parasites, etc.), as well as monitoring and eliminating abnormal cells within the body (such as cancer cells) and self-aging or damaged cells, thereby maintaining health and internal homeostasis of the organism.

As used in this application, "T cells" (T lymphocytes) are a type of lymphocyte originating from hematopoietic stem cells in the bone marrow, undergoing complex development in the thymus including positive selection and negative selection, eventually maturing and entering peripheral lymphoid organs and the bloodstream. They mainly include cytotoxic T cells (CD8⁺ T cells) and helper T cells (CD4⁺ T cells). Cytotoxic T cells are mainly responsible for recognizing and killing cells infected by viruses, intracellular parasites, and tumor cells. Through their cell-surface T cell receptor (TCR), they recognize antigen-major histocompatibility complex I (MHC-I) complexes on the target cell surface. After recognition, cytotoxic T cells release perforin and granzymes to induce apoptosis of the target cell. Activation of cytotoxic T cells requires TCR recognition of the antigen-MHC-I complex.

As used in this application, "stimulating T cells" or "activating T cells" means essentially the same, which refers to the T cells, after recognizing an antigen, becoming activated T cells (also called activated T lymphocytes), entering the cell cycle, and performing rapid proliferation, providing a sufficient number of effector T cells for the immune response.

As used in this application, "alleles" are a pair of genes located at the same position on a pair of homologous chromosomes, controlling a pair of corresponding traits. In human chromosomes, for a specific gene locus (the specific location of a gene on a chromosome), different gene forms may exist; these different forms are alleles. For example, in the cell DC0502 of this application, HLA-A0201 and HLA-A6801 are different gene forms at the HLA-A gene locus; thus HLA-A0201 and HLA-A6801 are alleles, whereas the specific HLA-A0201 is referred to as the HLA-A gene subtype.

As used in this application, "autologous" means a tissue, cell, or substance originating from the same individual; "allogeneic" means a tissue, cell, or substance originating from a different individual of the same species.

As used in this application, "HLA molecule-specific matching" means that a DC can activate T cells only when the HLA molecules of the DC are identical or similar to those of the T cells; or stated differently, a DC can activate only the T cells having HLA molecules that are identical or similar to its own. Notably, the "HLA molecule" here does not refer to all HLA molecules, but only to the HLA molecules that function in the specific T cell activation process.

As used in this application, "HLA-I class genes" refer to a category of major histocompatibility complex (MHC) genes that play a crucial role in immune response and recognition. The products encoded by "HLA-I class genes" are HLA-I molecules (also called HLA-I proteins). These molecules mainly participate in presenting endogenous antigens, assisting the immune system in recognizing self cells and pathogen-infected cells. Specifically, HLA-I molecules bind to antigen peptides synthesized within the cell, such as viral protein antigens synthesized in a cell after a virus infects the cell. After binding to the HLA-I class molecules, these antigen peptides are transported to the cell surface for recognition by CD8⁺ T lymphocytes. CD8⁺ T cells recognize these antigen peptide-HLA-I complexes through their T cell receptor (TCR), thereby initiating the cellular immune response and killing infected cells. "HLA-I class genes" mainly include three types, which are the HLA-A gene, HLA-B gene, and HLA-C gene, respectively, and their encoded products are HLA-A molecules, HLA-B molecules, and HLA-C molecules. The HLA-A molecules, HLA-B molecules, and HLA-C molecules differ in binding specificity to different antigen peptides. For example, HLA-A may have higher affinity for certain virus antigen peptides, while HLA-B may have a stronger ability to bind to other antigen peptides, depending on the structural characteristics of their antigen-binding grooves. The HLA-A gene, HLA-B gene, and HLA-C gene are highly polymorphic, with many different alleles present in the population. Different individuals carry different HLA-A genes, and the same individual may carry two different HLA-A alleles. A specific allele in an individual can be detected and classified using specific techniques, and specific alleles are called "gene subtypes". It is worth noting that, in this application, although "HLA-I class genes" include the three types of genes HLA-A gene, HLA-B gene, and HLA-C gene, it does not refer to all of the HLA-A gene, HLA-B gene, and HLA-C gene. Unless otherwise specified, "HLA-I class genes" can refer to any one or a combination of the HLA-A gene, HLA-B gene, and HLA-C gene. Those skilled in the art can make a judgment based on the context.

As used in this application, "peripheral blood mononuclear cell" (PBMC) refers to a cell in the peripheral blood that possesses a single nucleus, and these cells include lymphocytes (T cells, B cells, NK cells) and other monocytes. Monocytes can undergo a series of induced culture in vitro to obtain mature DCs, referred to as monocyte-derived DCs (MODC).

As used in this application, "knocking out" or "gene knockout", also called "gene knock-out" refers to removing a specific gene in the cell genome, or reducing its activity or expression. The "specific gene" is also called the "object gene" or "target gene".

As used in this application, "starting DC" refers to the starting cell used to prepare the DC; it can be a natural DC or an optimized DC, such as a DC capable of becoming immortalized after domestication or a DC capable of becoming immortalized after gene editing.

As used in this application, "immortalized" refers to possessing unlimited proliferation capacity. Under appropriate culture conditions, the immortalized cell can continuously perform cell division and uninterruptedly increase in cell number, unlike normal somatic cells which enter the senescence and death stage after a certain number of divisions.

As used in this application, "target antigen-specific T cell" refers to a T cell capable of recognizing a target antigen.

As used in this application, "negative expression" refers to the absence of the expected signal or marker in the cell, indicating that the detected target substance (such as protein, gene, antigen, etc.) has a content in very low amounts or not at all in the cell. For example, a DC with HLA-A gene negative expression means a DC with very low or no HLA-A molecule content.

As used in this application, CRISPR/Cas (Clustered Regularly Interspaced Short Palindromic Repeats/CRISPR-associated proteins) is a gene editing technique. Reference can be made to the instruction manual provided by the reagent supplier.

As used in this application, gRNA (guide RNA) is a key component in the CRISPR/Cas gene editing system, which is capable of guiding the Cas protein (e.g., Cas9) to the target DNA sequence, thereby realizing gene editing of specific gene locus.

As used in this application, "restricted antigen peptide" refers to an antigen peptide that, during an immune response, binds to a specific major histocompatibility complex (MHC) molecule and is presented by the MHC molecule to T lymphocytes for recognition. There is a certain restrictive relationship between these antigen peptides and the MHC molecule, meaning that only a specific antigen peptide can bind to a specific MHC molecule, and only a specific T-cell receptor (TCR) can recognize this peptide-MHC complex. Accordingly, the MHC molecule is referred to as the restricting MHC molecule for the antigen. For example, an HLA-A0201-restricted antigen peptide is a specific antigen peptide that binds to HLA-A0201, and HLA-A0201 is called the restricting HLA-A molecule for that peptide.

As used in this application, "antigen epitope" refers to the specific chemical group within an antigen molecule that determines the antigen specificity. For example, in this application, a CMV epitope is the specific region on the cytomegalovirus (CMV) antigen molecule that is recognized by immune cells; a Mart-1 epitope is the specific region on the melanoma-associated antigen (Mart-1 antigen) that is recognized by immune cells.

As used in this application, "reintroduction" means the reintroduction of a target gene. For example, reintroduction of HLA-A0201 refers to the introduction of the HLA-A0201 gene into a DC with *HLA-I* class genes knocked out by gene editing, viral, or mRNA gene introduction techniques, thereby causing it to re-express the HLA-A0201 molecule.

As used in this application, "HLA-ABC KO DC(s)" refers to DCs with negative expression of the HLA-A gene, HLA-B gene, and HLA-C gene (i.e., the DC does not produce the HLA-A molecule, HLA-B molecule, and HLA-C molecule). At the protein molecular level, these are also referred to as HLA-ABC-negative cells. Similarly, HLA-A0201 KO DC refers to a DC with negative expression of the HLA-A0201 gene (i.e., the DC does not produce the HLA-A0201 molecule).

As used in this application, "HLA-ABC⁺ DCs" refers to DCs with positive expression for at least one of the HLA-A gene, HLA-B gene, and HLA-C gene (i.e., at least one of the HLA-A molecule, HLA-B molecule, and HLA-C molecule is present in the DC).

As used in this application, flow cytometry (FCM) is a technology for performing rapid, quantitative analysis and separation of cells or particles (e.g., bacteria, viruses, etc.). Reference may be made to the instruction manual provided by the equipment or reagent supplier.

As used in this application, "gene-edited DC" refers to a DC that has undergone gene editing (UDC mix).

As used in this application, "UDC" refers to DCs that do not express HLA-I class molecules.

As used in this application, "UDC981 HLA-A0201" refers to UDC981 that expresses the HLA-A0201 molecule subtype.

### Embodiment 1

This embodiment provides a preparation method for a DC and the DC prepared thereby.

A preparation method for a DC, the method knocking out HLA-I class genes of a starting DC, the HLA-I class genes being HLA-A gene, HLA-B gene, and/or HLA-C gene; the DC screened for negative expression of the HLA-I class genes is the DC (UDC).

In this embodiment, the starting DC is DC0502, which is an immortalized DC deposited at the China General Microbiological Culture Collection Center (CGMCC), with a deposit time of December 25, 2024, deposit address of No. 3, Yard 1, Beichen West Road, Chaoyang District, Beijing, and deposit number of CGMCC No. 46259; the HLA-A gene subtype of this immortalized DC is HLA-A0201 and HLA-A6801; the HLA-B gene subtype is HLA-B0801 and HLA-B1507; the HLA-C gene subtype is HLA-C0303 and HLA-C0702.

In this embodiment, the HLA-I class genes are HLA-A gene, HLA-B gene and HLA-C gene, i.e., the HLA-A gene, HLA-B gene, and HLA-C gene of the knocked out starting DC.

In this embodiment, knockout of HLA-I class genes in the starting DC is performed using CRISPR/Cas gene editing to knockout the HLA-I class genes, the CRISPR/Cas gene editing including: designing one or more sgRNA that bind to the HLA-I class genes of the starting DC; using a CRISPR-Cas system to introduce the sgRNA into the starting DC to knock out the HLA-I class genes.

In the embodiment, a nucleotide sequence of the sgRNA is as shown in SEQ ID NO. 1 (CGGCTACTACAACCAGAGCG). This sgRNA can simultaneously target a pair of alleles of the HLA-A gene (HLA-A0201 and HLA-A6801), a pair of alleles of the HLA-B gene (HLA-B0801 and HLA-B1507), and a pair of alleles of the HLA-C gene (HLA-C0303 and HLA-C0702) of the starting DC, thereby simultaneously knocking out three pairs of alleles with extremely high gene-editing efficiency; the sgRNA was synthesized by GenScript.

In the embodiment, using the CRISPR-Cas system to introduce the sgRNA into the starting DC specifically includes:
Collecting DC0502 in logarithmic growth phase into a 15 mL centrifuge tube, centrifuging at 400 g for 5 min, discarding the supernatant, adding 10 mL phosphate-buffered saline (PBS), gently washing by inverting the centrifuge tube, and centrifuging at 400 g for 5 min;
Discarding the supernatant, using PBS to resuspend the cell pellet, and counting; according to a conventional electroporation protocol, preparing cell numbers at 1×10⁶ cells/test; centrifuging at 400 g for 5 min, discarding the supernatant, and using room temperature-equilibrated Gene Pulser Electroporation Buffer (purchased from BIO-RAD, Cat. No. 1652676) at 50 µL/test to resuspend the cell pellet, gently mixing, and transferring the cell suspension to a sterile 1.5 mL EP tube;
According to the instruction manual of GenCRISPR^{™} Cas9 v1.2 (purchased from GenScript, Cat. No. Z03702), adding GenCRISPR^{™} Cas9 v1.2 and sgRNA according to a molar ratio of 1:3 with electroporation buffer into the sterile EP tube; placing the mixture at room temperature to incubate for 30 min to form RNP complex;
Transferring the cell suspension from the EP tube into the RNP complex, mixing thoroughly, and transferring the mixture into a sterile electroporation cuvette;
Using BIO-RAD electroporation system: Gene Pulser Xcell Eukaryotic Electroporation System (Item No. 165-2661) to perform RNP complex electroporation with electroporation conditions of: pulse type: square wave; voltage: 200 V; pulse: 2 ms;
After electroporation completed, immediately transferring the cells into pre-warmed cell culture medium, the cell culture medium being XVIVO15 (LONZA, 04-418Q) + 400 IU/mL IL-2 (Sihuan Biopharma, NMPA Approval No. S10970015), placing the culture plate in a 37°C constant temperature incubator, and continuing to culture to obtain UDC mix.

In this embodiment, screening for DCs with negative expression of HLA-I class genes is performed by magnetic bead negative selection to obtain purified DCs with negative expression of HLA-I class genes, designated as HLA-ABC KO DC.

In this embodiment, Miltenyi Biotec MACS^{®} Separation system is used to perform cell separation, and operating procedures refer to the instruction manual of Anti-PE MicroBeads (Miltenyi Biotec, Order No. 130-048-801), and are specifically as follows:
Collecting cells of the cultured UDC mix after gene editing and after expansion, and labeling with primary antibody PE anti-human HLA-A,B,C Antibody (Biolegend, 311406); adding 5 µL of PE anti-human HLA-A,B,C Antibody per 100 µL of 1×10⁸ cells/mL cell suspension, mixing thoroughly, and incubating in a 4°C refrigerator in the dark for 30 min;
Washing the labeled cells, using Buffer prepared according to the instruction manual to resuspend the cell suspension, centrifuging at 300 g for 6 min, and washing once more;
Completely discarding the supernatant, adding 20 µL of Anti-PE MicroBeads per 1×10⁷ cells, mixing thoroughly, and placing in a 4°C refrigerator to incubate for 15 min;
Washing and centrifuging at 300 g for 6 min;
Completely discarding the supernatant, and using 500 µL of Buffer to resuspend the cell pellet;
Placing a magnetic rack in a biosafety cabinet, installing an LD separation column on the magnetic rack, and placing a collection tube under the separation column;
Before cell separation, adding 2 mL of Buffer to the separation column to fully wet the separation column, the Buffer passing through the separation column and flowing into the collection tube, and replacing the collection tube when the MACS Buffer has completely flowed;
Filtering the cell suspension labeled with Anti-PE MicroBeads using a 40 µm mesh to cause the cells to form a single-cell suspension;
Adding the filtered cell suspension into the separation column, and when drops flowing from the separation column almost stop, adding 2 mL of Buffer into the separation column again for washing, and collecting the cell suspension flowing into the collection tube as HLA-ABC KO DCs;
When the drops flowing from the separation column almost stop, removing the separation column from the magnetic rack and placing over a new collection tube, adding 3 mL of Buffer again into the separation column, connecting a plunger at an opening of the separation column, and pushing the liquid within the separation column into a new collection tube; the cell suspension is HLA-ABC⁺ DCs;
Seeding the separated HLA-ABC molecule-negative UDC into a cell culture plate, the cell culture medium being XVIVO15 (LONZA, 04-418Q) + 400 IU/mL IL-2 (Sihuan Biopharma, NMPAApproval No. S10970015), and placing the culture plate into a 37°C incubator to continue to culture.

This embodiment further provides performing identification of HLA-ABC KO DCs after magnetic bead separation.

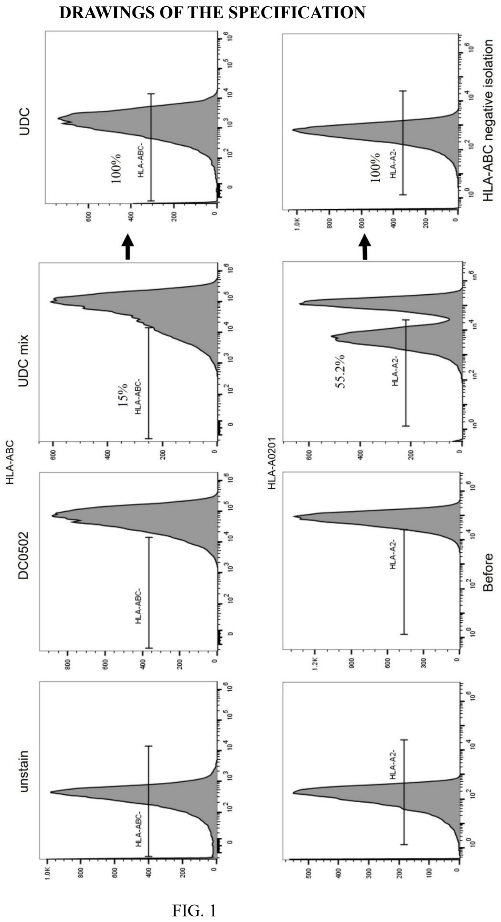

In this embodiment, flow cytometry was used to detect the proportion of HLA-ABC KO DC and HLA-A0201 KO DC in the DCs separated by magnetic beads, confirm the purity of HLA-ABC KO DC and HLA-A0201 KO DC. The detection results are shown in FIGURE 1.

As can be seen from the right side of the arrow in FIGURE 1, the gene-edited DCs after two rounds of negative selection by Anti-PE MicroBeads are 100% proportion of HLA-ABC KO DC and 100% proportion of HLA-A0201 KO DC. The purified gene-edited DCs are named as UDC.

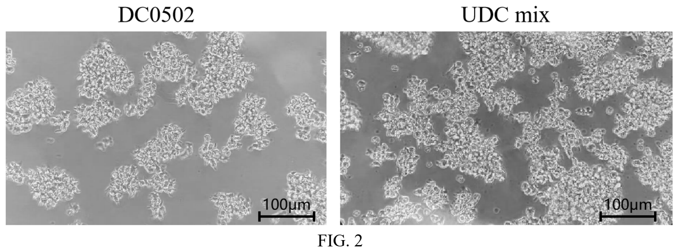

Cell morphology photographs of the DCs before and after gene editing are shown in FIGURE 2. It can be seen that the morphology of the gene-edited DCs (UDC mix) is consistent with that of wild-type DCs (DC0502).

### Embodiment 2

This embodiment provides detection of gene editing efficiency of the CRISPR/Cas gene editing method in Embodiment 1.

It is specifically detecting the efficiency of gene editing performed on the three genes HLA-A gene, HLA-B gene, and HLA-C gene (each gene containing two alleles, totaling 6 alleles) in DC0502.

In this embodiment, gene editing efficiency is detected by first using HLA-ABC antibody (Biolegend, 311406) to perform detection of overall HLA-ABC KO efficiency on the cultured UDC mix after electroporation, and then using HLA-A0201 antibody (BD Bioscience, 567739) to perform detection of HLA-A0201 single-gene editing efficiency on the cultured UDC mix obtained after electroporation, which specifically includes:

After DC0502 electroporation, culturing for 48 h, collecting the UDC mix after gene editing electroporation as well as DC0502 without gene editing performed into EP tubes, and performing staining for cell surface HLA-ABC molecules and HLA-A0201 molecules according to conventional staining procedures of flow cytometry.

The flow cytometry detection results are shown in FIGURE 1. As can be seen from the left side of the arrow in FIGURE 1, the cultured UDC mix after electroporation has 15% HLA-ABC molecule-negative, and 55.2% of HLA-A0201 are molecule-negative, indicating that the CRISPR/Cas gene editing method in Embodiment 1, particularly the designed targeted sgRNA, can simultaneously knock out the HLA-A gene, HLA-B gene, and HLA-C gene, and the gene editing efficiency is relatively high.

### Embodiment 3

This embodiment provides further validation and selection of monoclonals of the purified gene-edited DC (UDC).

The selection target in this embodiment is UDC monoclonals with complete knockout of the 3 pairs of alleles of the HLA-A gene, HLA-B gene, and HLA-C gene in a homozygous background genotype.

The purified gene-edited DCs were collected and plated into a 96-well plate according to a density of 0.5-1 cell/well to perform subclonal culture. The culture medium is XVIVO15 (LONZA, 04-418Q) + 400 IU/mL IL-2 (Sihuan Biopharma, NMPA Approval No. S10970015). After 2-3 weeks of culture and expansion, the expression of HLA-A molecules, HLA-B molecules, and HLA-C molecules, as well as the HLA-A gene, HLA-B gene, and HLA-C gene, were identified in the DC monoclonals.

From dozens of UDC monoclonals, seven monoclonals numbered 1-2, 1-3, 1-9, 1-10, 1-11, 1-19, and 1-20 were preferentially selected, and validation of HLA-A molecule, HLA-B molecule, and HLA-C molecule expression was performed using the flow cytometry antibodies PE anti-human HLA-A (BD Bioscience, 567739), Alexa Fluor^{®} 647 anti-human HLA-B (BD Bioscience, 569662), and FITC anti-human HLA-ABC (Biolegend, 311404). Flow cytometry sample preparation was performed according to conventional staining operating procedures of flow cytometry, analysis was performed using a flow cytometer (Beckman), and data were plotted using FlowJo software (BD).

At the same time, genomic DNA was extracted from the cells, and the sequences of the HLA-A gene, HLA-B gene, and HLA-C gene were detected by a sequencing method.

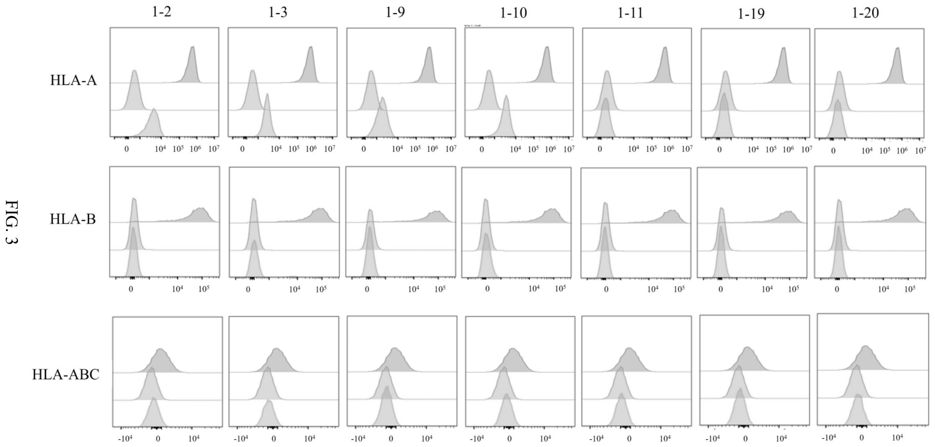

The results are shown in FIGURE 3 to FIGURE 5.

FIGURE 3 shows overlaid histograms of flow cytometry analysis of purified gene-edited DCs (UDCs) selected for complete knockout of the 3 pairs of alleles of the HLA-A gene, HLA-B gene, and HLA-C gene in a homozygous background. Wherein, the first row histogram in each group of histograms is the DC0502 negative control, the second row histogram in each group of histograms is the UDC mix positive control, and the third row histogram in each group of histograms is the HLA-I class molecule expression intensity of each monoclonal, wherein the third row of the first group corresponds to HLA-A molecule expression intensity, the third row histogram of the second group corresponds to HLA-B molecule expression intensity, and the third row histogram of the third group corresponds to HLA-ABC molecule expression intensity. The results show that among the 7 monoclonals 1-2, 1-3, 1-9, 1-10, 1-11, 1-19, and 1-20, the HLA-A molecule, HLA-B molecule, and HLA-ABC molecule of the monoclonals 1-11, 1-19, and 1-20 are background level.

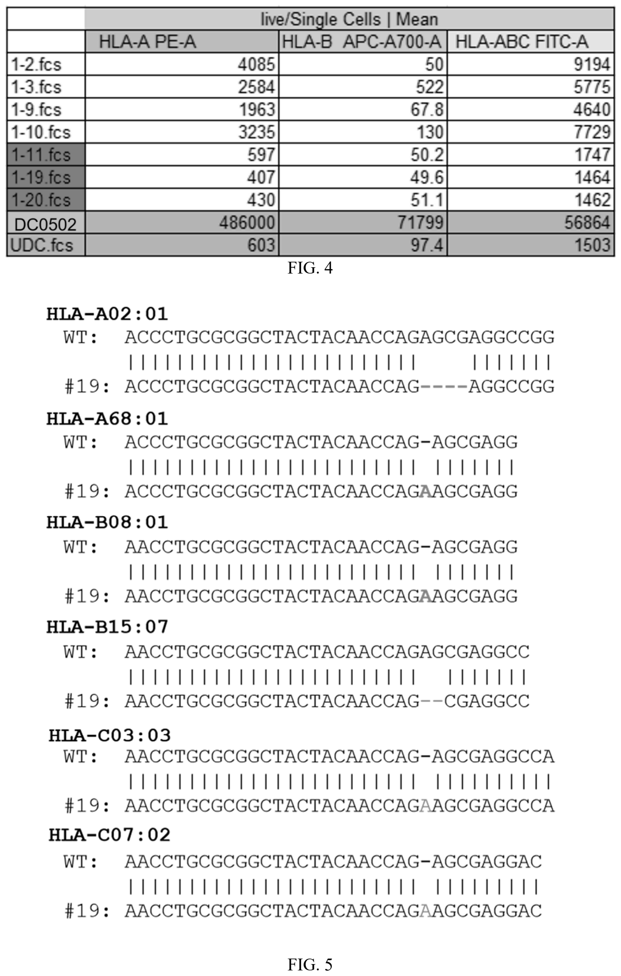

As shown in FIGURE 4, through analysis of mean fluorescence intensity of the HLA-A molecule, HLA-B molecule, and HLA-ABC molecule, the HLA-A molecule, HLA-B molecule, and HLA-ABC molecule of the monoclonals 1-11, 1-19, and 1-20 have negative expression.

Sequencing of the UDC monoclonals 1-19 was performed, and the results are shown in FIGURE 5:

Nucleotide sequence of a partial segment of the HLA-A0201 gene is: ACCCTGCGCGGCTACTACAACCAGAGCGAGGCCGG (SEQ ID NO. 8); after gene editing, the nucleotide sequence of this segment is: ACCCTGCGCGGCTACTACAACCAGAGGCCGG (SEQ ID NO. 9);

Partial nucleotide sequence of the HLA-A6801 gene is:
ACCCTGCGCGGCTACTACAACCAGAGCGAGG (SEQ ID NO. 10); after gene editing, the nucleotide sequence of this segment is: ACCCTGCGCGGCTACTACAACCAGAAGCGAGG (SEQ ID NO. 11);

Partial nucleotide sequence of the HLA-B0801 gene is:
AACCTGCGCGGCTACTACAACCAGAGCGAGG (SEQ ID NO. 12); after gene editing, the nucleotide sequence of this segment is: AACCTGCGCGGCTACTACAACCAGAAGCGAGG (SEQ ID NO. 13);

Partial nucleotide sequence of the HLA-B1507 gene is:
AACCTGCGCGGCTACTACAACCAGAGCGAGGCC (SEQ ID NO. 14); after gene editing, the nucleotide sequence of this segment is: AACCTGCGCGGCTACTACAACCAGCGAGGCC (SEQ ID NO. 15);

Partial nucleotide sequence of the HLA-C0303 gene is:
AACCTGCGCGGCTACTACAACCAGAGCGAGGCCA (SEQ ID NO. 16); after gene editing, the nucleotide sequence of this segment is: AACCTGCGCGGCTACTACAACCAGAAGCGAGGCCA (SEQ ID NO. 17);

Partial nucleotide sequence of the HLA-C0702 gene is:
AACCTGCGCGGCTACTACAACCAGAGCGAGGAC (SEQ ID NO. 18); after gene editing, the nucleotide sequence of this segment is: AACCTGCGCGGCTACTACAACCAGAAGCGAGGAC (SEQ ID NO. 19);

Indicating that the 3 pairs of alleles of the HLA-A gene, HLA-B gene, and HLA-C gene were completely knocked out, and are HLA-ABC KO DC.

The above flow cytometry and sequencing results prove that the UDC monoclonals 1-19 are homozygous with complete knockout of the 3 pairs of alleles of the HLA-A gene, HLA-B gene, and HLA-C gene. The preferentially selected UDC monoclonals 1-19 were named UDC981 and deposited at the China General Microbiological Culture Collection Center (CGMCC), with a deposit time of December 25, 2024, deposit address of No. 3, Yard 1, Beichen West Road, Chaoyang District, Beijing, and deposit number of CGMCC No. 46260.

### Embodiment 4

This embodiment provides detection of proliferation capacity of the starting DC (DC0502) and UDC monoclonals 1-19 (UDC981).

DC0502 and UDC981 were separately collected and counted, and seeded into T25 culture flasks according to a cell density of 5×10⁵ cells/mL (initial total cell number: 5×10⁶ cells), and 10 mL of culture medium XVIVO15 (LONZA, 04-418Q) + 400 IU/mL IL-2 (Sihuan Biopharma, NMPAApproval No. S10970015) was added. The culture medium color and cell status were observed daily. After the culture medium turned yellow, half the volume was replaced, and samples were taken and counted, and at the same time, fresh medium was added to adjust the cell density to 5-8×10⁵ cells/mL. Passaging and counting were continued for 30 days.

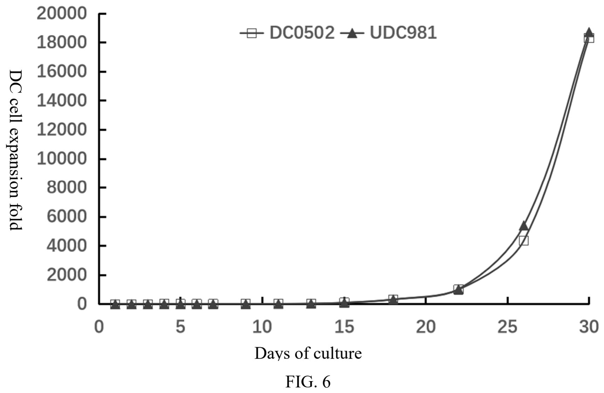

The expansion culture results of DC0502 and UDC981 cells are shown in FIGURE 6. After 30 days of culture, the expansion fold reached up to 18,000-fold, indicating that DC0502 and UDC981 can be continuously passaged and expansion cultured, possessing immortalized characteristics.

### Embodiment 5

This embodiment provides detection and identification of cell phenotypes of the UDC monoclonals 1-19 (UDC981).

Identification of phenotypes of UDC981 cells is performed by comprehensive evaluation of several aspects, such as expression of lineage markers on the DC surface, DC maturation markers, and functionally related molecules such as antigen-presenting molecules, costimulatory molecules, and T cell activation-related molecules.

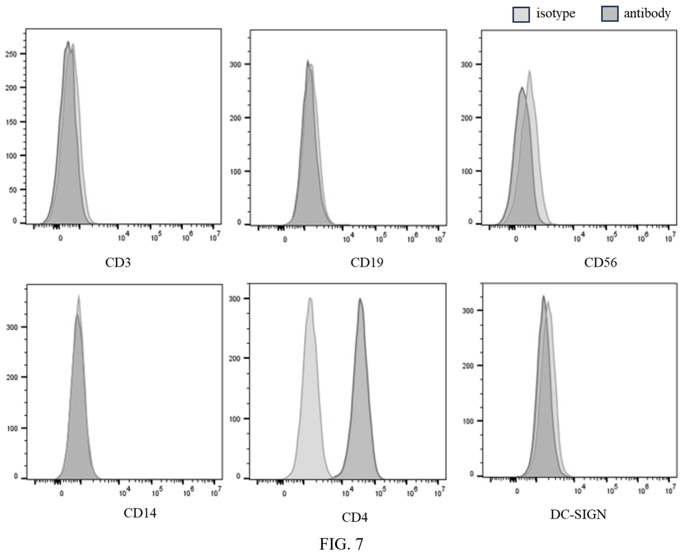

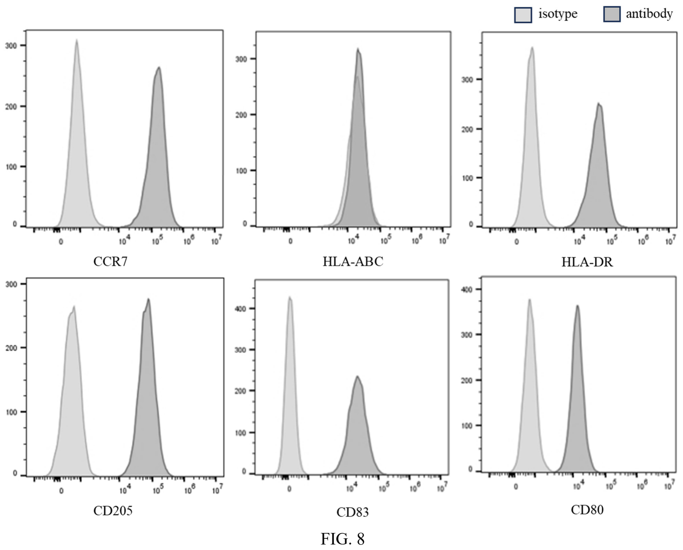

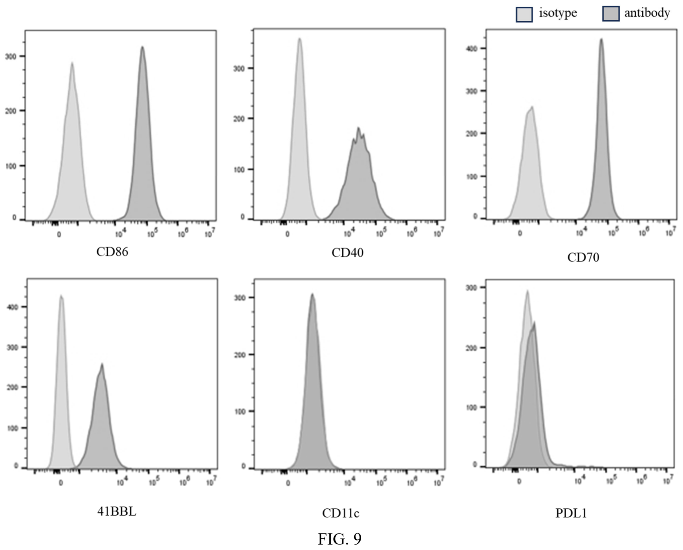

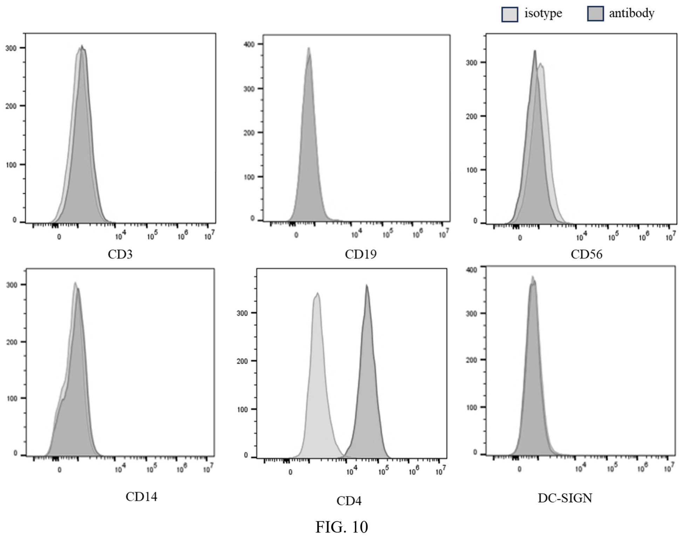

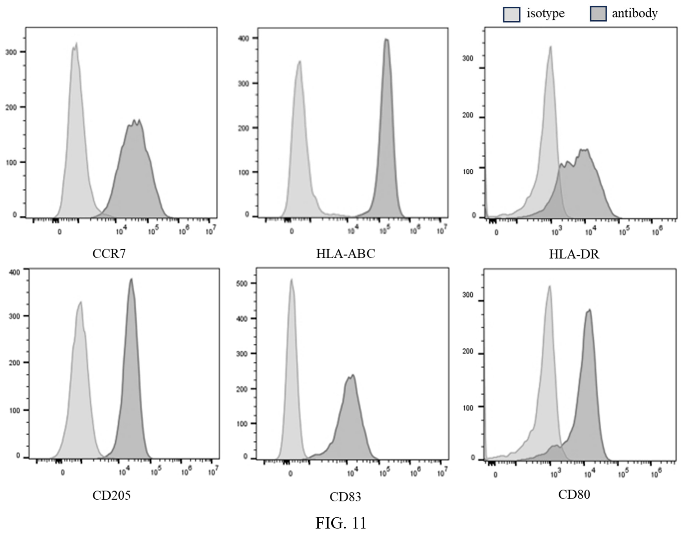

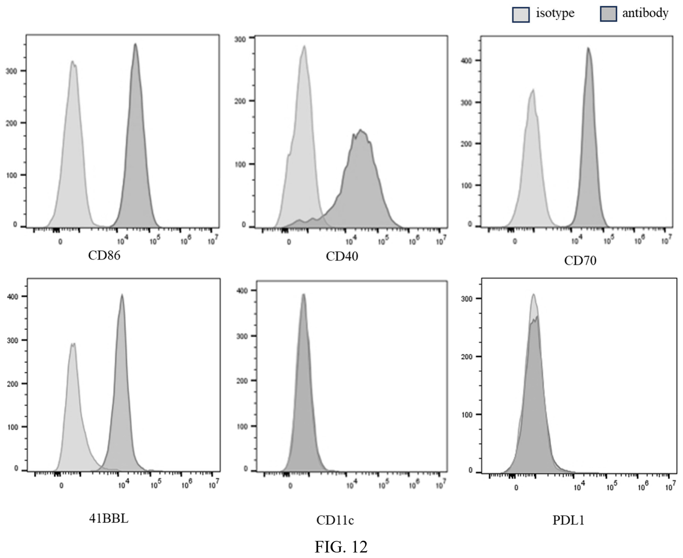

Comprehensive expression detection of phenotypes and costimulatory molecules of UDC981 was performed to validate the influence of gene editing on DC phenotypes and expression of related functional molecules. The specific steps are as follows:
Collecting UDC monoclonal UDC981 and DC0502 cells separately into 1.5 mL EP tubes, performing flow cytometry sample preparation according to conventional staining operating procedures of flow cytometry, using a flow cytometer (Beckman) to perform analysis, and using FlowJo software (BD) to plot data. Antibody reagents used are: PE anti-human CD4 (Biolegend, 300441), FITC anti-human CD19 (Biolegend, 363008), PE anti-human CD56 (Biolegend, 362508), BV421 anti-human CD14 (Biolegend, 325628), FITC anti-human CD4 (Biolegend, 300506), FITC anti-human DC-SIGN (Biolegend, 330103), PE/Cyanine7 anti-human CCR7 (Biolegend, 353226), Alexa Fluor700 anti-human HLA-ABC (Biolegend, 311438), BV510 anti-human HLA-DR (Biolegend, 307646), PE anti-human CD205 (Biolegend, 342204), PE/Cyanine7 anti-human CD83 (Biolegend, 305330), BV510 anti-human CD80 (Biolegend, 305234), PE anti-human CD86 (Biolegend, 305406), BV650 anti-human CD40 (Biolegend, 334338), PE anti-human CD70 (Biolegend, 355104), PE/Cyanine7 anti-human CD137L (Biolegend, 311512), FITC anti-human CD11C (Biolegend, 301604), PE anti-human CD274 (Biolegend, 153611), as well as corresponding isotype controls (Biolegend). Results are shown in FIGURE 7 to FIGURE 12.

As shown in FIGURE 7 to FIGURE 12, comparison of phenotypes and expression intensities of costimulatory molecules between UDC981 and DC0502 validates that after the DCs undergo gene editing knockout of the HLA-A gene, HLA-B gene, and HLA-C gene encoding HLA-I class molecules, the classical antigen-presenting HLA-I class molecules HLA-A molecule, HLA-B molecule, and HLA-C molecule are not expressed, while other DC surface molecules maintain the same phenotype and costimulatory molecule expression capacity as that of DC WT (DC0502).

### Embodiment 6

This embodiment provides expression of HLA-I class molecules in UDC monoclonals 1-19 (UDC981) after reintroduction of genes encoding HLA-I class molecules.

Antigen-presenting capacity is one of the core functions of DCs as professional antigen-presenting cells. DCs use classical HLA-I class molecules (HLA-A molecule, HLA-B molecule, or HLA-C molecule) to present endogenous antigens to TCR on the surface of CD8⁺ T cells to recognize and bind to, thereby stimulating CD8⁺ T cell activation and initiating specific immune cell responses. UDC981, with knockout of genes expressing the HLA-A molecule, HLA-B molecule, and HLA-C molecule, does not express the HLA-A molecule, HLA-B molecule, and HLA-C molecule, and does not possess intrinsic antigen-presenting capacity. Through various methods of gene introduction pathways to reintroduce different types of genes encoding HLA-I class molecules, whether the stimulation, activation, and proliferation of antigen-specific cells matching different HLA-I class subtypes can be achieved is validated.

In this embodiment, reintroducing different HLA-I class molecules into UDC981 includes: on the basis of UDC981, using a lentiviral method to separately integrate genes encoding different HLA-I class molecules into the UDC981 genome.

In this embodiment, based on several of the most common human HLA-A molecule subtypes, separate construction of UDCs reintroduced with HLA-A0201, HLA-A1101, and HLA-A2402 is selected. The amino acid sequences of HLA-A0201, HLA-A1101, and HLA-A2402 molecules are shown in SEQ ID NO. 2 to SEQ ID NO. 4, and their encoding genes were entrusted to GenScript for synthesis:
HLA-A0201 molecule: 






  (SEQ ID NO. 2);
HLA-A1101 molecule: 
MAVMAPRTLLLLLSGALALTQTWAGSHSMRYFYTSVSRPGRGEPRFIAVGYVDD
TQFVRFDSDAASQRMEPRAPWIEQEGPEYWDQETRNVKAQSQTDRVDLGTLRGYYNQSE
DGSHTIQIMYGCDVGPDGRFLRGYRQDAYDGKDYIALNEDLRSWTAADMAAQITKRKWE
AAHAAEQQRAYLEGRCVEWLRRYLENGKETLQRTDPPKTHMTHHPISDHEATLRCWALGF
YPAEITLTWQRDGEDQTQDTELVETRPAGDGTFQKWAAVVVPSGEEQRYTCHVQHEGLPK
PLTLRWELSSQPTIPIVGIIAGLVLLGAVITGAVVAAVMWRRKSSDRKGGSYTQAASSDSAQG
SDVSLTACKV (SEQ ID NO. 3);
HLA- A2402 molecule:







 (SEQ ID NO. 4).

As an example, using a lentiviral method to separately integrate genes encoding different HLA-I class molecules into the UDC981 genome includes viral vector construction and lentiviral packaging, which specifically includes:
Inserting genes encoding HLA-A0201, HLA-A1101, and HLA-A2402 molecules into a commercial lentiviral vector pCDH-CMV-MCS-EF1, transforming into Stbl3 competent cells, and after sequencing for confirmation, using an endotoxin-free plasmid extraction kit to extract and purify plasmids;
Culturing 293T cells in 10 cm cell culture dishes, and when confluence does not exceed 80%, starting to perform plasmid transfection; mixing target plasmid, packaging plasmids psPAX2 and pMD2.G, and transfection reagent polyethylenimine (PEI) at a mass ratio of 3:2:1 in serum-free DMEM culture medium; then, adding the mixture containing lentiviral plasmids encoding HLA-A0201, HLA-A1101, or HLA-A2402 to 293T cells, culturing at 37°C, ultracentrifuging cell culture supernatants collected at 48 h and 72 h to concentrate and collect virus particles carrying the target gene, and performing viral titer determination;
Collecting UDC981 cells and counting, and plating cells into 4 wells of a 6-well plate according to 5×10⁵ cells/mL, with quantitatively measured cell suspension added at 2 mL/well; separately infecting each UDC981 experimental well with lentiviruses containing genes encoding HLA-A0201, HLA-A1101, or HLA-A2402 according to MOI=50; after the infected UDC981 undergo 3 weeks of culture and expansion, performing HLA-ABC-positive cell separation using the Miltenyi MACS^{®} Separation system, with the operating procedures referring to the Anti-PE MicroBeads (Miltenyi Biotec, Order No. 130-048-801) instruction manual.

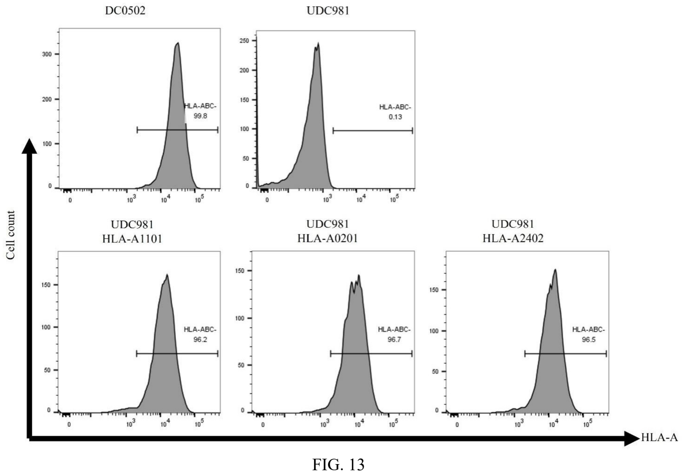

Results are shown in FIGURE 13. As seen in FIGURE 13, after positive selection, purified UDC981 HLA-A0201, UDC981 HLA-A1101, and UDC981 HLA-A2402 with HLA-A positivity rates of 95% were obtained.

The above results indicate that after reintroduction of genes encoding HLA-I class molecules into UDC981, it can re-express HLA-I class molecules.

### Embodiment 7

This embodiment validates the antigen-presenting capacity of UDC981 HLA-A0201, i.e., validates whether it can stimulate proliferation of antigen-specific T cells matched to the HLA-A0201 molecule.

Stimulating proliferation of antigen-specific T cells with the HLA-A0201 molecule subtype includes:
Loading UDC981 HLA-A0201 with antigen peptides restricted to the HLA-A0201 molecule subtype, an antigen epitope of the antigen peptides being an antigen epitope recognized by target antigen-specific T cells;
Co-culturing the DCs loaded with antigen peptides with a T cell population to activate and expand specific T cells capable of recognizing the above-described antigen epitope.

In this embodiment, the T cell population is PBMC (Bokang Bioengineering (Shandong) Co., Ltd.) with HLA-A subtype HLA-A0201, and is thawed prior to use. In this embodiment, thawing includes: taking out PBMC rested for 2 h from the incubator, centrifuging at 600 g for 5 min, discarding the supernatant, adding Optivitro (EXCEL, TE000-N022) + 2% Clin-SFM (Beijing Clin-SFM Biotechnology Co., Ltd., 1007) culture medium into the centrifuge tubes to resuspend the cells, adjusting the cell suspension density to 2×10⁶ cells/mL, and seeding into a 24-well plate at 1 mL/well.

In this embodiment, UDC981 HLA-A0201 is the UDC in which HLA-A0201 was introduced, as described in Embodiment 6.

In this embodiment, the antigen peptides are CMV epitope and Mart-1 epitope, which are antigen peptides restricted to the HLA-A molecule.

In this embodiment, loading the antigen peptides onto UDC981 HLA-A0201 includes:
Separately adding cell suspensions of DC0502 (positive control), UDC981 (negative control), and UDC981 HLA-A0201 into centrifuge tubes, centrifuging at 600 g for 5 min, discarding the supernatant, using culture medium to separately resuspend each tube, the cell culture medium being XVIVO15 (LONZA, 04-418Q) + 400 IU/mL IL-2 (Sihuan Biopharma, NMPA Approval No. S10970015), adjusting the cell suspension density to 1.0×10⁶ cells/mL, and seeding into a 48-well plate, with the cells of each of the DC0502, UDC981, and UDC981 HLA-A0201 cell types plated in 4 wells; separately adding CMV-A0201 and Mart-1-A0201 antigen peptides (amino acid sequences shown in SEQ ID NO. 5 and SEQ ID NO. 6, synthesized by GenScript, 10 µg/µL) and blank controls according to 1:1000 to each culture well, and placing the cell culture plates in a constant temperature incubator at 37°C to incubate for 4 h to obtain three types of DCs loaded with antigen peptides.

In this embodiment, co-culturing the DCs loaded with antigen peptides with the T cell population to stimulate proliferation of antigen-specific T cells includes:
Taking different DCs loaded with antigen peptides and corresponding blank control DCs and counting, adding the DCs into PBMC culture wells according to DC:PBMC=1:100, gently mixing, and placing the DC/PBMC co-culture plate into a 5% CO₂ incubator at 37°C to culture for approximately 14 days, performing medium replenishment or expansion operations every 2 days.

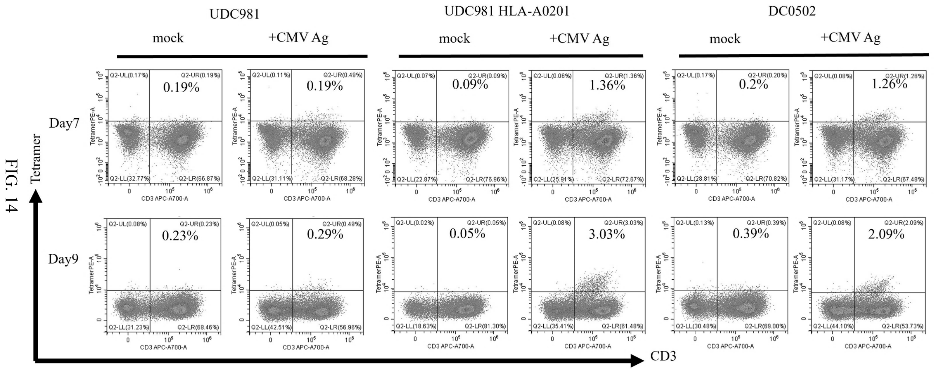

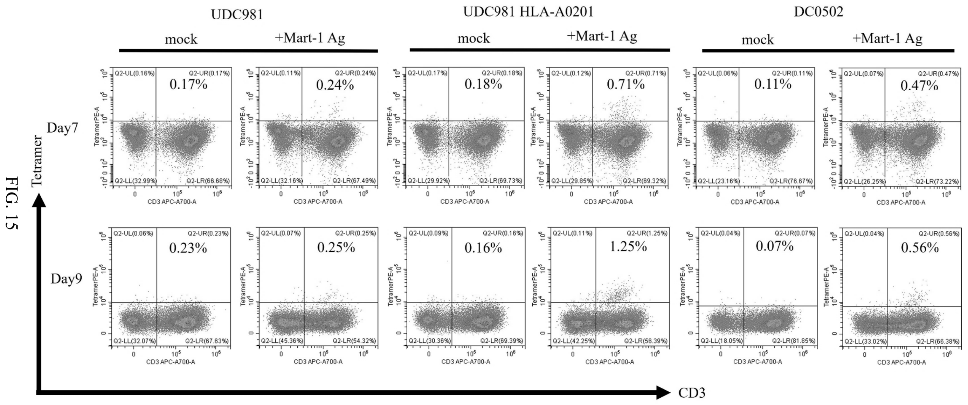

During the culture process, detection of the proportion of antigen-specific T cell proliferation stimulated by UDC981 HLA-A0201 loaded with antigen peptides was performed, the detection method including:
On day 7 and day 9 of the co-culture of DC loaded with antigen peptides and PBMC, collecting cells from different sample groups, separately adding into 1.5 mL EP tubes, and centrifuging at 400 g for 5 min to collect cells;
Discarding the supernatant, using 50 µL of FACS buffer (purchased from Beijing Tianjingsha Gene Technology Co., Ltd.) to resuspend the cell pellet, adding Human TruStain FcX into the sample tubes at 1 µL/EP tube, and incubating at room temperature in the dark for 15 min;
After incubation was completed, adding corresponding Tetramer-CMV-A0201 or Tetramer-Mart-1 A0201 to each tube at 0.5 µL Tetramer/EP tube according to the different loaded antigen peptide, and incubating at 37°C in the dark for 30 min;
After incubation was completed, adding 200 µL of FACS buffer to wash the cells, centrifuging at 400 g for 5 min to collect the cells, discarding the supernatant, and performing flow cytometry sample preparation according to conventional staining procedures of flow cytometry; the PBMC surface marker molecule antibodies used are: Alexa Fluor 700 anti-human CD3, APC anti-human CD4, Brilliant Violet 510^{™} anti-human CD8, and PE/Cyanine7 anti-human CD56 (NCAM);
Using a flow cytometer (Beckman) to perform analysis, and Beckman CytExpert software to analyze flow cytometry data, results are shown in FIGURE 14 and FIGURE 15, wherein, FIGURE 14 is with CMV-A0201 antigen added, FIGURE 15 is with Mart-1-A0201 antigen added, and statistical results are shown in Table 1 and Table 2.

**Table 1: Antigen peptide CMV-tetramer -positive proportions (%)**

| DC type | Day 7 | Day 9 |
|---|---|---|
| UDC981 | 0 | 0 |
| UDC981 HLA-A0201 | 1.27 | 2.98 |
| DC0502 | 1.06 | 1.7 |

**Table: 2: Antigen peptide Mart-1-tetramer -positive proportions (%)**

| DC type | Day7 | Day9 |
|---|---|---|
| UDC981 | 0 | 0 |
| UDC981 HLA-A0201 | 0.53 | 1.09 |
| DC0502 | 0.36 | 0.49 |

As can be seen from FIGURE 14 and FIGURE 15 in combination with Table 2 and Table 3:
The co-culture of UDC981 (negative control) with PBMC at time points of day 7 and day 9 did not stimulate antigen peptide CMV-tetramer -positive or antigen peptide Mart-1-tetramer - positive specific CD8⁺ T cells. This is because UDC981 lacks HLA-A molecules and does not possess antigen-presenting capacity, and the results are as expected;
The co-culture of DC0502 (HLA-A0201-positive) with PBMC at time points of day 7 and day 9 stimulated CMV-tetramer -positive specific CD8⁺ T cells at proportions of 1.06% and 1.7%, and Mart-1-tetramer -positive specific CD8⁺ T cells at proportions of 0.36% and 0.49%;
The co-culture of UDC981 HLA-A0201 with PBMC at time points of day 7 and day 9 stimulated CMV-tetramer -positive specific CD8⁺ T cells at proportions of 1.27% and 2.98%, and Mart-1-tetramer -positive specific CD8⁺ T cells at proportions of 0.53% and 1.09% (calculation method: tetramer -positive proportion in group with antigen peptide added - tetramer -positive proportion in Mock group).

The above embodiment indicates that UDC981 HLA-A0201 can stimulate different types of antigen-specific CD8⁺ T cells in the same manner as wild-type DC0502 (HLA-A0201-positive), with positivity rates gradually increasing with time from day 7 to day 9, proving that after reintroducing the HLA-A0201 molecule on the basis of UDC981, UDC981 HLA-A0201 possesses antigen-presenting capacity consistent with that of wild-type DC0502 (HLA-A0201) and its effect is even better than that of wild-type DC0502.

### Embodiment 8

This embodiment validates the antigen-presenting capacity of UDC981 HLA-A2402, i.e., validates whether it can stimulate activation and proliferation of antigen-specific T cells matched to the HLA-A2402 molecule.

Referring to Embodiment 7, in this embodiment, the T cell population is PBMC (Bokang Bioengineering (Shandong) Co., Ltd.) with HLA-A subtype HLA-A2402, and is thawed prior to use. In this embodiment, thawing includes: taking out PBMC rested for 2 h from the incubator, centrifuging at 600 g for 5 min, discarding the supernatant, adding Optivitro (EXCEL, TE000-N022) + 2% Clin-SFM (Beijing Clin-SFM Biotechnology Co., Ltd., 1007) culture medium into the centrifuge tubes to resuspend the cells, adjusting the cell suspension density to 2×10⁶ cells/mL, and seeding into a 24-well plate at 1 mL/well.

In this embodiment, UDC981 HLA-A2402 is the UDC in which HLA-A2402 was introduced, as described in Embodiment 6. The HLA-A gene subtype of the starting DC (DC0502) of UDC981 HLA-A2402 is HLA-A0201 and HLA-A6801, meaning that an HLA-A gene subtype different from that of the starting DC is introduced into the UDC.

In this embodiment, the antigen peptide is a CMV epitope, which is an antigen peptide restricted to HLA-A2402.

In this embodiment, loading the antigen peptides onto UDC981 HLA-A2402 includes:
Separately adding cell suspensions of the company-constructed DC-0801 and DC-0802 (both containing HLA-A2402 and HLA-A1101 molecule phenotypes, positive controls) and UDC981 HLA-A2402 into centrifuge tubes, centrifuging at 600 g for 5 min, discarding the supernatant, using cell culture medium to separately resuspend each tube, the cell culture medium being XVIVO15 (LONZA, 04-418Q) + 400 IU/mL IL-2 (Sihuan Biopharma, NMPA Approval No. S10970015), adjusting the cell suspension density to 1.0×10⁶ cells/mL, and seeding into a 48-well plate, with the cells of each of the DC-0801, DC-0802, and UDC981 HLA-A2402 cell types plated into 3 culture wells; separately adding CMV-A2402 antigen peptide (amino acid sequence shown in SEQ ID NO. 7, synthesized by GenScript, 10 µg/µL) and blank controls according to 1:1000 to each culture well, and placing the cell culture plates in a constant temperature incubator at 37°C to incubate for 4 h to obtain four types of DCs loaded with antigen peptides.

In this embodiment, co-culturing the DCs loaded with antigen peptides with the T cell population to stimulate proliferation of antigen-specific T cells includes:
Taking different DCs loaded with antigen peptides and corresponding blank control DCs and counting, adding the DCs into PBMC culture wells according to DC:PBMC=1:100, gently mixing, and placing the DC/PBMC co-culture plate into a 5% CO₂ incubator at 37°C to culture for approximately 14 days, performing medium replenishment or expansion operations every 2 days.

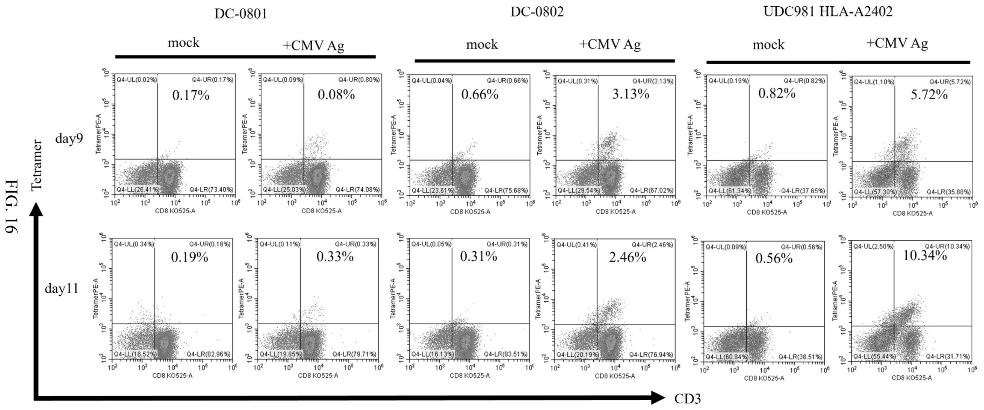

During the culture process, detection of the proportion of antigen-specific T cell proliferation stimulated by UDC981 HLA-A0201 loaded with antigen peptides was performed, the detection method including:
On day 9 and day 11 of the co-culture of DC loaded with antigen peptides and PBMC, collecting cells from different sample groups, separately adding into 1.5 mL EP tubes, and centrifuging at 400 g for 5 min to collect cells;
Discarding the supernatant, using 50 µL of FACS buffer to resuspend the cell pellet, adding Human TruStain FcX into the sample tubes at 1 µL/EP tube, and incubating at room temperature in the dark for 15 min;
After incubation was completed, adding corresponding Tetramer-CMV-A2402 to each tube at 0.5 µL Tetramer/EP tube according to the loaded antigen peptide, and incubating at 37°C in the dark for 30 min;
After incubation was completed, adding 200 µL of FACS buffer to wash the cells, centrifuging at 400 g for 5 min to collect the cells, discarding the supernatant, and performing flow cytometry sample preparation according to conventional staining procedures of flow cytometry; the PBMC surface marker molecule antibodies used are: Alexa Fluor 700 anti-human CD3, APC anti-human CD4, Brilliant Violet 510^{™} anti-human CD8, and PE/Cyanine7 anti-human CD56 (NCAM);
Using a flow cytometer (Beckman) to perform analysis, and Beckman CytExpert software to analyze flow cytometry data, results are shown in FIGURE 16, and statistical results are shown in Table 3.

**Table 3: Antigen peptide CMV-tetramer -positive proportions (%)**

| DC type | Day 9 | Day 11 |
|---|---|---|
| UDC981HLA-A2402 | 4.9 | 9.78 |
| DC-0801 | 0.63 | 0.15 |
| DC-0802 | 2.47 | 2.15 |

As can be seen from FIGURE 16 and Table 3:

The co-culture of the two wild-type DCs containing the HLA-A2402 subtype (DC-0801 and DC-0802) with PBMC at time points of day 9 and day 11 stimulated antigen peptide CMV-tetramer -positive specific CD8⁺ T cells at proportions of 0.63%, 0.15%, and 2.47%, 2.15%, respectively;
The co-culture of UDC981 HLA-A2402 with PBMC at time points of day 9 and day 11 stimulated antigen peptide CMV-tetramer -positive specific CD8⁺ T cells at proportions of 4.9% and 9.78%, and the proportion of antigen peptide CMV-tetramer -positive specific CD8⁺ T cells approximately doubled with time from day 9 to day 11, demonstrating that UDC981 possesses extremely strong antigen-presenting capacity after reintroduction of HLA-A2402.

In this experiment, the stimulation capacity of UDC981 HLA-A2402 on antigen-specific CD8⁺ T cells was significantly stronger than that of wild-type DCs (HLA-A2402 subtype) DC-0801 and DC-0802, possibly because UDC981 HLA-A2402 is a single subtype of HLA-A2402, whereas DC-0801 and DC-0802 are heterozygous for HLA-A2402 and HLA-A1101, making the expansion of antigen-specific T cells by UDC981 HLA-A2402 more specific.

### Embodiment 9

This embodiment validates the immunogenic response of UDC981 HLA-A11 to allogeneic PBMC.

T cells are stimulated to proliferate when in contact with allogeneic somatic cells with mismatched HLA-I. To investigate whether single-allele HLA-I matching can reduce immune stimulation of allogeneic T cells, the immunogenicity of UDC981 reintroduced with HLA-I molecules was evaluated through T cell proliferation experiments. The details are as follows: allogeneic PBMC (Bokang Bioengineering (Shandong) Co., Ltd.) possessing single-allele HLA-A*11:01 but mismatched at other alleles were labeled with CFSE. The staining process was: PBMC with HLA-A subtype HLA-A02:HLA-A11 (Bokang Bioengineering (Shandong) Co., Ltd.) were thawed prior to use. Thawing includes: taking out PBMC rested for 2 h from the incubator, centrifuging at 600 g for 5 min, discarding the supernatant, adding Optivitro (EXCEL, TE000-N022) + 2% Clin-SFM (Beijing Clin-SFM Biotechnology Co., Ltd., 1007) culture medium into the centrifuge tubes to resuspend the cells, and adjusting the cell suspension density to 1×10⁷ cells/mL. CFSE Cell Division Tracker Kit (Biolegend, GYS0004) was added to the cell suspension to a final concentration of 5 µM; another portion of cells was taken and not stained to serve as a control. The stained cell suspension was placed in a 37°C incubator and incubated in the dark for 15 min.

Five times the volume of pre-warmed culture medium (1640+10% FBS) was added to the stained cell suspension and incubated for 5 min, and excess free dye was removed.

The cell suspension was centrifuged at 600 g for 10 min, the cell pellet was resuspended using pre-warmed culture medium (1640+10% FBS+20 IU IL-2), and the cell density was adjusted to 1×10⁶ cells/mL. The stained and unstained PBMC were seeded into a 6-well plate with 2 mL cell suspension/well added. Wild-type DC0502 with HLA-A subtype HLA-A02:HLA-A68 was added to the CFSE-labeled PBMC culture wells at a ratio of 200:1, and UDC981 HLA-A11 (specifically HLA-A1101, with amino acid sequence as shown in SEQ ID NO. 3, encoding gene entrusted to GenScript for synthesis, with UDC981 HLA-A11 construction referring to Embodiment 6) was added to another well; PBMC culture wells alone (CFSE-labeled and unlabeled) were set up, and a positive control well was set up by adding 2 µL T Cell TransAct^{™} (Miltenyi Biotec, 130-128-758).

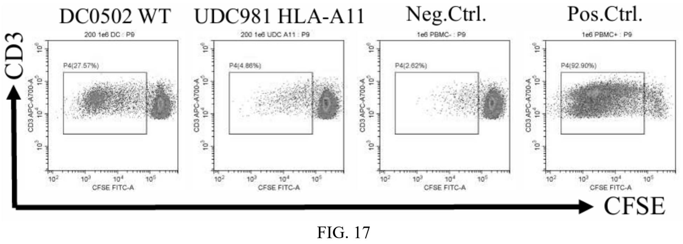

After 6 days of co-culture, CFSE fluorescence intensity in the CD3⁺ T cell subpopulation was detected by flow cytometry (FACS). Analysis was performed using a flow cytometer (Beckman), and flow cytometry data were analyzed using Beckman CytExpert software. Results are shown in FIGURE 17.

The results show that the percentage of proliferating CD3⁺ T cells in the wild-type DC0502 group (DC0502 WT) with mismatched HLA-I subtype was as high as 27.57%; in the UDC981 HLA-A11 group, this percentage decreased to 4.86%, which was relatively close to the background level (unstimulated CD3⁺ cells, 2.62%). This indicates that single-allele HLA-I matching UDC981 can reduce non-antigen-specific immune stimulation of allogeneic T cells.

From the above-described contents, the DC and the preparation method and application thereof provided by this application can make DCs modified by gene editing to not express HLA-A, HLA-B, and HLA-C molecules. Sequencing results indicate that in the obtained UDC monoclonals, the 6 alleles of HLA-A, HLA-B, and HLA-C all have mutations at the corresponding sites. The modified UDC can re-express any subtype of HLA-I class molecule through gene editing, viral vector, or mRNA techniques, overcoming the intrinsic HLA-I class molecule restriction of DCs, which can serve as an APC to expand antigen-specific T cells restricted to any HLA-I. At the same time, allogeneic immunogenicity testing of UDC981 after reintroduction of HLA-I molecules proves that UDC981 in practical applications possesses both antigen-presenting and specific immune activation functions, while avoiding the core risk of allogeneic immune rejection. The low non-specific T cell activation characteristics of UDC981 cells provide key evidence for achieving safe allogeneic universal application. Combined with antigen peptide loading or mRNA antigen expression techniques, it can be developed into DC vaccine products targeting any HLA-I class.

## Claims

1. A dendritic cell (DC), **characterized in that**, *HLA-I* class genes of the DC are knocked out, the *HLA-I* class genes comprising an *HLA-A* gene, an *HLA-B* gene, and/or an *HLA-C* gene.

2. The DC according to claim 1, **characterized in that**, the *HLA-I* class genes comprise the *HLA-A* gene, the *HLA-B* gene, and the *HLA-C* gene.

3. The DC according to claim 1 or 2, **characterized in that**, the DC is an immortalized DC.

4. The DC according to claim 3, **characterized in that**, the immortalized DC is UDC981, deposited at China General Microbiological Culture Collection Center (CGMCC), with a deposit time of December 25, 2024, deposit address of No. 3, Yard 1, Beichen West Road, Chaoyang District, Beijing, and deposit number of CGMCC No. 46260.

5. A cell population of a dendritic cell (DC), **characterized in that**, the cell population comprises any one of the DCs according to claims 1-4.

6. A preparation method for a dendritic cell (DC), **characterized in that**, the preparation method comprises the following steps:
knocking out *HLA-I* class genes of a starting DC, the *HLA-I* class genes comprising an *HLA-A* gene, an *HLA-B* gene, and/or an *HLA-C* gene; knocking out *HLA-I* class genes of a starting DC comprising: using homologous recombination, CRISPR/Cas gene editing, or transcription activator-like effector nuclease to knock out the *HLA-I* class genes of the starting DC;
the DC screened for negative expression of the *HLA-I* class genes is the DC.

7. The preparation method for a DC according to claim 6, **characterized in that**, the CRISPR/Cas gene editing comprises the following steps:
designing one or more sgRNA that bind to the *HLA-I* class genes of the starting DC;
using a CRISPR-Cas system to introduce the sgRNA into the starting DC to knock out the *HLA-I* class genes.

8. The preparation method for a DC according to claim 7, **characterized in that**, the *HLA-A* gene subtype of the starting DC is *HLA-A0201* and/or *HLA-A6801;* and/or the *HLA-B* gene subtype is *HLA-B0801* and/or *HLA-B1507;* and/or the *HLA-C* gene subtype is *HLA-C0303* and/or *HLA-C0702*; a nucleotide sequence of the sgRNA is as shown in SEQ ID NO. 1.

9. The preparation method for a DC according to any one of claims 6-8, **characterized in that**, the starting DC is an immortalized DC.

10. The preparation method for a DC according to claim 9, **characterized in that**, the immortalized DC is DC0502, deposited at China General Microbiological Culture Collection Center (CGMCC), with a deposit time of December 25, 2024, deposit address of No. 3, Yard 1, Beichen West Road, Chaoyang District, Beijing, and deposit number of CGMCC No. 46259.

11. A method for expanding target antigen-specific T cells, **characterized in that**, the method comprises:
obtaining an HLA-I class molecule subtype of a T cell population that is restricted by a target antigen, the *HLA-I* class molecule comprising an *HLA-A* molecule, an *HLA-B* molecule, and/or an *HLA-C* molecule;
introducing an *HLA-I* class gene encoding the HLA-I class molecule subtype into the DCs according to claims 1-4;
loading the DCs introduced with the *HLA-I* class gene with an antigen peptide that is restricted to the *HLA-I* class molecule subtype, an antigen epitope of the antigen peptide being an antigen epitope recognized by the target antigen-specific T cell;
co-culturing the DCs loaded with the antigen peptide with the T cell population, thereby activating and expanding specific T cells capable of recognizing the antigen epitope.

12. The method for expanding target antigen-specific T cells according to claim 11, **characterized in that**, the antigen peptide is derived from a tumor or a virus.

13. The method for expanding target antigen-specific T cells according to claim 11 or 12, **characterized in that**, the method of introducing *HLA-I* class genes comprises: gene editing, a virus, or mRNA method.

14. The method for expanding target antigen-specific T cells according to claim 13, **characterized in that**, the T cell population comprises PBMC.

15. A reagent for expanding target antigen-specific T cells, **characterized in that**, the reagent comprises any one of the DCs of claims 1-4 or the DC cell population of claim 5.

16. The reagent according to claim 15, **characterized in that**, the reagent further comprises: an *HLA-I* class gene encoding an *HLA-I* class molecule subtype of the target antigen-specific T cells, the *HLA-I* class molecule comprising an *HLA-A* molecule, an *HLA-B* molecule, and/or an *HLA-C* molecule.

17. A modified DC, **characterized in that**, original *HLA-I* class genes in the modified DC are knocked out, and at the same time has exogenous *HLA-I* class genes introduced, the exogenous *HLA-I* class genes being *HLA-I* class genes of a single subtype, the exogenous *HLA-I* class genes comprising an *HLA-A* gene, an *HLA-B* gene, or an *HLA-C* gene; the original *HLA-I* class genes at least comprise the same type of *HLA-I* class gene as the exogenous *HLA-I* class gene.

18. An sgRNA, **characterized in that**, a nucleotide sequence of the sgRNA is as shown in SEQ ID NO. 1.

19. An application of the sgRNA according to claim 18 in knocking out *HLA-I* class genes of cells, **characterized in that**, the *HLA-I* class genes comprise an *HLA-A* gene, an *HLA-B* gene, and/or an *HLA-C* gene; a subtype of the *HLA-A* gene of the cell is *HLA-A0201* and/or *HLA-A6801;* and/or a subtype of the *HLA-B* gene is *HLA-B0801* and/or *HLA-B1507;* and/or a subtype of the *HLA-C* gene is *HLA-C0303* and/or *HLA-C0702.*

20. The application according to claim 19, **characterized in that**, the cells comprise dendritic cells (DCs) or T cells.

21. The application according to claim 20, **characterized in that**, the DC is an immortalized DC.

22. The application according to claim 21, **characterized in that**, the immortalized DC is DC0502, deposited at China General Microbiological Culture Collection Center (CGMCC), with a deposit time of December 25, 2024, deposit address of No. 3, Yard 1, Beichen West Road, Chaoyang District, Beijing, and deposit number of CGMCC No. 46259.

23. An immortalized DC, named DC0502, deposited at China General Microbiological Culture Collection Center (CGMCC), with a deposit time of December 25, 2024, deposit address of No. 3, Yard 1, Beichen West Road, Chaoyang District, Beijing, and deposit number of CGMCC No. 46259.
